# EUROPÄISCHE PATENTSCHRIFT

(11) **EP 2 978 858 B1**
(45) Veröffentlichungstag und Bekanntmachung des Hinweises auf die Patenterteilung: **19.06.2019**
(21) Anmeldenummer: 14711284.1
(22) Anmeldetag: 20.03.2014
(51) Int. Cl.: C12Q 1/6881, C12Q 1/6883

(54) **IN VITRO-VERFAHREN ZUR PROGNOSTISCHEN BEURTEILUNG DER ERFOLGSAUSSICHTEN EINER IMPLANTATION UND/ODER TRANSPLANTATION**
IN VITRO METHOD FOR THE PROGNOSIS OF SUCCESSFUL IMPLANTATION AND/OR TRANSPLANTATION
PROCÉDÉ IN VITRO D'ÉVALUATION PRONOSTIQUE DES CHANCES DE RÉUSSITE D'UNE IMPLANTATION ET/OU TRANSPLANTATION

(30) Priorität: 27.03.2013 DE 102013205516; 28.01.2014 DE 102014201528
(43) Veröffentlichungstag der Anmeldung: 03.02.2016
(73) Patentinhaber: TETEC Tissue Engineering Technologies AG, 72770 Reutlingen (DE)
(72) Erfinder: MOLLENHAUER, Jürgen, 72764 Reutlingen (DE); GAISSMAIER, Christoph, 72770 Reutlingen (DE); BENZ, Karin, 73037 Göppingen (DE)
(74) Vertreter: Patentanwälte Ruff, Wilhelm, Beier, Dauster & Partner mbB
(86) Internationale Anmeldenummer: PCT/EP2014/055667
(87) Internationale Veröffentlichungsnummer: WO 2014/154569

(56) Entgegenhaltungen:
- WO-A1-2011/127561
- WO-A2-2008/061804
- DE-A1- 10 243 131
- BERNSTEIN P ET AL: "Expression pattern differences between osteoarthritic chondrocytes and mesenchymal stem cells during chondrogenic differentiation", OSTEOARTHRITIS AND CARTILAGE, BAILLIERE TINDALL, LONDON, GB, Bd. 18, Nr. 12, 1. Dezember 2010 (2010-12-01), Seiten 1596-1607, XP027523834, ISSN: 1063-4584 [gefunden am 2010-09-29]
- BARLIC ARINA ET AL: "Quantitative analysis of gene expression in human articular chondrocytes assigned for autologous implantation", JOURNAL OF ORTHOPAEDIC RESEARCH, Bd. 26, Nr. 6, Juni 2008 (2008-06), Seiten 847-853, XP002723044, ISSN: 0736-0266
- CAMERON TREVOR L ET AL: "Global comparative transcriptome analysis of cartilage formation in vivo", BMC DEVELOPMENTAL BIOLOGY, BIOMED CENTRAL LTD., LONDON, GB, Bd. 9, Nr. 1, 10. März 2009 (2009-03-10), Seite 20, XP021047634, ISSN: 1471-213X, DOI: 10.1186/1471-213X-9-20
- KARLSSON C ET AL: "Genome-wide expression profiling reveals new candidate genes associated with osteoarthritis", OSTEOARTHRITIS AND CARTILAGE, BAILLIERE TINDALL, LONDON, GB, Bd. 18, Nr. 4, 1. April 2010 (2010-04-01), Seiten 581-592, XP026976185, ISSN: 1063-4584 [gefunden am 2010-01-04]
- ASHWELL M S ET AL: "Gene expression profiling of chondrocytes from a porcine impact injury model", OSTEOARTHRITIS AND CARTILAGE, BAILLIERE TINDALL, LONDON, GB, Bd. 16, Nr. 8, 1. August 2008 (2008-08-01) , Seiten 936-946, XP022849044, ISSN: 1063-4584, DOI: 10.1016/J.JOCA.2007.12.012 [gefunden am 2008-02-13]
- AGARWAL S ET AL: "242 TRANSCRIPTOME-WIDE ANALYSIS OF ARTICULAR CHONDROCYTES EXPOSED TO BIOMECHANICAL FORCES OF VARIOUS MAGNITUDES", OSTEOARTHRITIS AND CARTILAGE, BAILLIERE TINDALL, LONDON, GB, Bd. 16, 1. September 2008 (2008-09-01), Seiten S111-S112, XP025689954, ISSN: 1063-4584, DOI: 10.1016/S1063-4584(08)60287-3 [gefunden am 2008-09-01]
- STOOP REINOUT ET AL: "Comparison of marker gene expression in chondrocytes from patients receiving autologous chondrocyte transplantation versus osteoarthritis patients", ARTHRITIS RESEARCH AND THERAPY, BIOMED CENTRAL, LONDON, GB, vol. 9, no. 3, 27 June 2007 (2007-06-27), page R60, XP021031084, ISSN: 1478-6354, DOI: 10.1186/AR2218

## Beschreibung

### ANWENDUNGSGEBIET UND STAND DER TECHNIK

Die vorliegende Erfindung betrifft ein in vitro-Verfahren zur prognostischen Beurteilung der Erfolgsaussichten einer Implantation und/oder Transplantation.

Die Erstellung von Genexpressionsprofilen oder die Analyse des Transkriptoms haben sich mit der Etablierung der Microarray-Technologie zu einem wichtigen Werkzeug in der biomedizinischen Wissenschaft etabliert.

Insbesondere die Entwicklung von RNA-Sequenzierungsmethoden der zweiten Generation (Next Generation Sequencing, NGS) hat nicht nur zu einer drastischen Senkung der Kosten für die Durchführung einer Transkriptomanalyse geführt, sondern auch die Genauigkeit bei der Identifizierung bisher unbekannter Genaktivitäten erhöht. Beispiele für Anwendungsbereiche von Genexpressionsprofilen sind die Diagnostik und Prognostik von Krankheiten, die Nachsorgeanalyse von Therapien, die Analyse genetischer Prädispositionen, die Untersuchung pharmakologischer Wirkmechanismen sowie die qualitative und quantitative Untersuchung von Wachstums- und Differenzierungsprozessen von Zellen und Geweben.

Ein gebräuchliches Verfahren zur Auswertung von Genexpressionsdaten stellt die Differenz-Analyse dar, mit der sowohl die Expression bekannter Gene untersucht wird als auch der Nachweis unbekannter Gene erfolgen kann. Bei diesem Verfahren werden die Expressionsdaten der zu untersuchenden Probe mit dem Genexpressionsmuster von Referenzproben oder auch mit den Expressionsdaten ausgewählter Gene abgeglichen bzw. verglichen. Im Fall der Untersuchung der Expression von pathophysiologisch relevanten Genen werden beispielsweise die Expressionsdaten von gesundem Gewebe (Referenzprobe) mit den Expressionsdaten von erkranktem Gewebe (Messprobe) wie zum Beispiel Tumorgewebe verglichen. Basierend auf diesem Vergleich lassen sich Aussagen zur qualitativen (Ja/Nein-Antwort) oder zur quantitativen Expression (Erhöhung oder Verringerung der Expression) ausgewählter Gene treffen und diese wiederum einem bestimmten Zustand, beispielsweise einem pathologischen Zustand, zuordnen.

In der DE 10 2010 033 565 A1 werden diverse Marker zur in vitro Bestimmung der pharmazeutischen Identität, Reinheit oder Potenz von Chondrozyten (Knorpelzellen) offenbart, mittels derer sich die Chondrozyten auf ihre Eignung für eine voraussichtlich erfolgreiche Chondrozytentransplantation überprüfen lassen. Die Etablierung dieser Marker wurde von der Tatsache getragen, dass Chondrozyten hinsichtlich ihrer Eignung, als autologe Zellen für eine Implantation zur Knorpelregeneration eingesetzt zu werden, stark variieren können, und zwar nicht nur Chondrozyten eines Spenders im Verhältnis zu Chondrozyten eines anderen Spenders, sondern auch Chondrozyten desselben Spenders. Ferner wurde berücksichtigt, dass die Kultivierung von Chondrozyten deren Eigenschaften derart verändern kann, dass sie für eine Implantation nicht mehr so geeignet sind, wie direkt nach der Isolierung aus dem Spender.

Aus der DE 102 43 131 A1 ist ein Verfahren zur Identifizierung einer Chondrozytenkultur, die in der Lage ist, als Knorpeltransplantat zu dienen, sowie ein Verfahren zum Bestimmen des Expressionsstatus eines oder mehrerer Gene in einer Chondrozytenkultur bekannt.

Gegenstand der WO 2011/127561 A1 ist ein Verfahren zur Beurteilung von Erfolgsaussichten bei Patienten mit Lungenfibrose, bei welchem das Genexpressionsniveau diverser Gene bestimmt wird.

Ferner ist bekannt, dass Unterschiede im Phänotyp sowie in der Genexpression zwischen primären humanen Chondrozyten und mesenchymalen Stammzellen während der chondrogenen Differenzierung in dreidimensionalen Pellet-Kulturen existieren (P. Bernstein et al.: "Expression pattern differences between osteoarthritic chondrocytes and mesenchymal stem cells during chondrogenic differentiation", Osteoarthritis and Cartilage (2010), 18, Seiten 1596-1607).

Ferner wurde eine Marker-Genexpression in Chondrozyten von Patienten, welche eine autologe Chondrozytentransplantation erhalten hatten, mit einer Marker-Genexpression in Chondrozyten von an Arthrose erkrankten Patienten verglichen. Hierzu wurde das Genexpressionsprofil von Collagen Typ I, Collagen Typ II, Collagen Typ X, Aggrecan, IL-1β sowie Aktivin-ähnlicher Kinase-1 bestimmt (Stoop et al.: "Comparison of marker gene expression in chondrocytes vom patients receiving autologous chondrocyte transplantation versus osteoarthritis patients", Arthritis Research & Therapy (2007) 9/3: R60).

Aus der WO 2008/061804 A2 ist ein in vitro-Verfahren zur Bestimmung der Fähigkeit einer Zellpopulation zur Produktion von stabilem hyalinem Knorpel bekannt. Das Verfahren basiert auf der Bestimmung der Expression eines Sets aus zumindest drei Markergenen, wobei das Set FRZB, ALK1 und einen oder mehrere Marker ausgewählt aus der Gruppe bestehend aus PEDF, COL11, COL2, FGFR3, OPN, BMP-2 und RASF-PLA aufweist.

Obgleich eine selektive Analyse einiger weniger, insbesondere in den zellulären Stoffwechsel involvierter, Gene durchaus zu leistungsstarken Ergebnissen bei der Qualitätssicherung von zu transplantierenden Zellen führen kann, sind die Ergebnisse eines solchen Vorgehens in ihrer statistischen Aussagekraft dennoch beschränkt, zumal die chondrozytäre Differenzierung lediglich einen Parameter für die Beurteilung eines Heilerfolges darstellt.

### AUFGABE UND LÖSUNG

Vor diesem Hintergrund lag daher der vorliegenden Erfindung die Aufgabe zugrunde, ein Verfahren bereitzustellen, welches aus dem Stand der Technik bekannte Unzulänglichkeiten umgeht und insbesondere eine validere Individualprognostik erlaubt.

Diese Aufgabe wird gelöst durch ein in vitro-Verfahren mit den Merkmalen des unabhängigen Anspruchs 1. Bevorzugte Ausführungsformen des Verfahrens sind in den abhängigen Ansprüchen 2 bis 5 angegeben. Der Wortlaut sämtlicher Ansprüche wird hiermit durch ausdrückliche Bezugnahme zum Inhalt der Beschreibung gemacht.

Weiterhin wird ein in vitro-Verfahren zur prognostischen Beurteilung bzw. Prognose der Geweberegenerationsfähigkeit und/oder der zellulären Potenz und/oder der Erfolgsaussichten einer Implantation, vorzugsweise Zellimplantation, und/oder Transplantation, insbesondere zur prognostischen Beurteilung bzw. Prognose eines Implantat- und/oder Transplantatversagens, offenbart.

Das Verfahren zeichnet sich besonders dadurch aus, dass das Transkriptom von Zellen, insbesondere von Zellen eines Patienten (Patientenzellen), und/oder die auf das Transkriptom zurückgehende bzw. auf dem Transkriptom basierende Genexpression von Zellen, insbesondere von Zellen eines Patienten (Patientenzellen), in vitro analysiert werden/wird.

Bei der Transkriptomanalyse und/oder der Analyse einer auf dem Transkriptom beruhenden Genexpression können/kann mit besonderem Vorteil das Transkriptions- und/oder Translationsverhalten aller Gene einer Zelle erfasst werden und nicht nur - wie aus dem Stand der Technik bekannt - die Transkription bzw. Translation einiger weniger, insbesondere anhand ihrer Bedeutung für den zellulären Stoffwechsel, gezielt ausgewählter Gene. Mittels der vorgesehenen Transkriptomanalyse kann insbesondere ein vollständiges metabolisches Profil, ausgedrückt in der Genexpressionsaktivität der betreffenden Zellen, vorzugsweise Patientenzellen, erstellt werden.

Überraschenderweise stellte sich nun heraus, dass den im Rahmen der Transkriptomanalyse von Patientenzellen gewonnenen Transkriptom- und/oder Genexpressionsprofilen eine therapeutische Wertigkeit hinsichtlich der Geweberegenerationsfähigkeit sowie der Erfolgsaussichten einer implantologischen und/oder transplantologischen Maßnahme bei dem bzw. den betreffenden Patienten zugeordnet werden kann. Dies konnte von den Erfindern im Rahmen einer retrospektiven klinischen Nachbeobachtung am Beispiel einer matrix- bzw. trägergestützten autologen Chrondozytentransplantation (MACT) erfolgreich verifiziert werden.

Da die erhaltenen Profile auf einer in vitro-Analyse des Transkriptoms und/oder einer auf dem Transkriptom beruhenden Genexpression beruhen, ist eine im Vergleich zu gattungsgemäßen Verfahren validerer Einzelprognostik, d.h. eine Einzelprognostik mit höherer statistischer Aussagekraft, möglich.

Mit anderen Worten wird ein Verfahren zum Prognostizieren der Geweberegeneration, der zellulären Potenz, eines Implantationserfolges oder -misserfolges und/oder eines Transplantationserfolges oder -misserfolges offenbart.

Der Ausdruck "Zellimplantation" bezieht sich im Sinne der vorliegenden Offenbarung auf eine Implantation unter Verwendung eines mit Zellen beladenen bzw. inokulierten Implantats.

Der Ausdruck "Transkriptom" umfasst im Sinne der vorliegenden Offenbarung wenigstens die Summe der zu einem bestimmten Zeitpunkt in einer Zelle von der DNA in mRNA (messenger RNA oder Boten-RNA) umgeschriebenen Gene. Bevorzugt umfasst der Ausdruck "Transkriptom" im Sinne der vorliegenden Offenbarung jedoch die Summe der zu einem bestimmten Zeitpunkt in einer Zelle von DNA in RNA umgeschriebenen Gene, also die Gesamtheit aller in einer Zelle hergestellten RNA-Moleküle.

Der Ausdruck "Transkriptomprofil" (bzw. Transkriptommuster) bezeichnet im Sinne der vorliegenden Offenbarung das Profil (bzw. Muster) aller vorzugsweise mittels hybridisierungsbasierter und/oder sequenzbasierter Methoden, insbesondere Sequenzmethoden der zweiten Generation, erfassbaren Transkripte der Zellen.

Der Ausdruck "Genexpression" umfasst im Sinne der vorliegenden Erfindung die im Verlauf der Transkription stattfindende Synthese von RNA, insbesondere von mRNA (primäres Genprodukt), regulativer RNA und/oder weiteren RNA-Typen, und/oder die auf reifer mRNA-Sequenzen aufbauende Translation zu Proteinen (sekundäre Genprodukte). Als Beispiele für regulative RNA seien microRNA (miRNA), Small interfering RNA (siRNA) und/oder kleine nukleäre RNA (small nuclear RNA) genannt. Beispiele für die in diesem Absatz zudem erwähnten weiteren RNA-Typen stellen ribosomale RNA (rRNA) und/oder transfer-RNA (tRNA) dar, welche ebenfalls zu den primären Genprodukten gerechnet werden.

Der Ausdruck "Genexpressionsprofil" (bzw. Genexpressionsmuster) bezeichnet im Sinne der vorliegenden Erfindung die Interpretation der vorzugsweise mittels hybridisierungsbasierter und/oder sequenzbasierter Methoden, insbesondere Sequenzierungsmethoden der zweiten Generation, generierten Daten als Profil (bzw. Muster) der Genaktivitäten der untersuchten Zellen.

Der Ausdruck "Geweberegeneration" kann im Sinne der vorliegenden Offenbarung grundsätzlich die Regeneration jedweden Körper- bzw. Patientengewebes umfassen. Bevorzugt umfasst der Ausdruck "Geweberegeneration" jedoch die Stützgeweberegeneration, bevorzugt Knorpelge-weberegeneration, besonders bevorzugt Gelenkknorpelregeneration, und/oder Bandscheibengeweberegeneration .

Entsprechend kann der Ausdruck "Geweberegenerationsfähigkeit" im Sinne der vorliegenden Offenbarung grundsätzlich die Fähigkeit zur Regeneration jedweden Körper- bzw. Patientengewebes umfassen, insbesondere die Fähigkeit zur Stützgeweberegeneration, bevorzugt Knorpelgeweberegeneration, besonders bevorzugt Gelenkknorpelregeneration, und/oder Bandscheibengeweberegeneration.

Unter dem Ausdruck "zelluläre Potenz" soll im Sinne der vorliegenden Offenbarung die Fähigkeit von Gewebezellen verstanden werden, gewebespezifische Eigenschaften zu entwickeln und/oder die Entwicklung gewebespezifischer Eigenschaften aufrechtzuerhalten bzw. wiederaufzunehmen, insbesondere nach einer vorangegangenen in vitro-Kultivierung. Beispielsweise soll unter der Potenz von Chondrozyten deren Fähigkeit verstanden werden, extrazelluläre Matrix zu produzieren und/oder die Produktion von extrazellulärer Matrix wiederaufzunehmen, insbesondere wenn die Chondrozyten in eine zu behandelnde Defektstelle implantiert werden.

Der Ausdruck "matrix- oder trägergestützte Zellimplantation" bzw. "matrix- oder trägergestützte Zelltransplantantion" bedeutet im Sinne der vorliegenden Erfindung die Implantation bzw. Transplantation eines mit autologen Zellen versehenen bzw. inokulierten Implantats.

Die Zellen können grundsätzlich menschlichen und/oder tierischen Ursprungs sein. Mit anderen Worten kann es sich bei den Zellen um menschliche und/oder tierische Zellen handeln.

Bevorzugt stammen die Zellen von einem menschlichen Patienten.

Insbesondere kann es sich bei den Zellen um körpereigene oder autologe Zellen handeln.

Bevorzugt werden die Zellen einem Patienten in Form einer Gewebeprobe entnommen. Abhängig von der Art oder Herkunft der Probe kann es vorteilhaft sein, die Probe vor Durchführung der Transkriptomanalyse aufzubereiten. Eine geeignete Aufbereitung der Probe kann Schritte wie Zentrifugieren, Aufkonzentrieren, Homogenisieren, in vitro Vermehrung sowie weitere dem Fachmann grundsätzlich bekannte Aufbereitungsschritte umfassen.

In einer bevorzugten Ausführungsform stammen die Zellen von einem Patientengewebe, dessen Regenerationsfähigkeit und/oder dessen zelluläre Potenz beurteilt werden soll.

Insbesondere stammen die Zellen von einem Patientengewebe mit einem Defekt, der mittels der Implantation und/oder Transplantation behandelt werden soll.

Bevorzugt stammen die Zellen von einem Stützgewebe, weiter bevorzugt Knorpelgewebe, besonders bevorzugt Gelenkknorpelgewebe, und/oder Bandscheibengewebe.

In einer weiteren Ausführungsform handelt es sich bei den Zellen um Stützgewebezellen, bevorzugt Chondrozyten (Knorpelzellen), und/oder Vorläuferzellen davon, besonders bevorzugt Gelenkchondrozyten, Bandscheibenzellen, insbesondere Nukleus- und/oder Annuluszellen, und/oder Vorläuferzellen davon.

Bei den Zellen handelt es sich in einer weiteren Ausführungsform um gesunde Zellen bzw. um Zellen, welche aus gesunden Gewebeteilen bzw. -arealen stammen.

Bei der Implantation im Sinne der vorliegenden Erfindung handelt es sich in einer besonders bevorzugten Ausführungsform um eine matrix- oder trägergestützte autologe Zellimplantation, bevorzugt matrix- oder trägergestützte autologe Chondrozytenimplantation (matrix-assisted autologous chondrocyte implantation, MACI). Geeignete Matrices stellen insbesondere Kollagenträger dar. Eine bevorzugte Matrix bzw. ein bevorzugter Kollagen-Träger ist ein schichtförmig aufgebautes Implantat aus einer Perikardmembran und einem Kollagenschwamm, wobei der Kollagenschwamm vorzugsweise säulenförmige Poren besitzt, welche senkrecht oder im Wesentlichen senkrecht zur Perikardmembran orientiert sind und sich mittels einseitiger Lyophilisierung ausbilden lassen. Ein derartiger Kollagenträger wird von der Anmelderin beispielsweise unter der Bezeichnung Novocart® Basic bzw. Novocart® 3D kommerziell vertrieben. Bezüglich weiterer Merkmale und Vorteile eines solchen Kollagen-Trägers wird zudem auf die EP 1 824 420 B1 Bezug genommen, deren Offenbarungsgehalt in Bezug auf das darin beschriebene Implantat zur Reparatur eines Knorpeldefektes hiermit durch ausdrückliche Bezugnahme zum Inhalt der vorliegenden Beschreibung gemacht wird.

In einer weiteren Ausführungsform handelt es sich bei der Transplantation im Sinne der vorliegenden Erfindung um eine autologe Zelltransplantation, bevorzugt autologe Chondrozytentransplantation.

Bevorzugt werden die Zellen vor Durchführung der Transkriptomanalyse und/oder der Analyse der auf das Transkriptom zurückgehenden Genexpression in vitro kultiviert und insbesondere vermehrt. Die Kultivierung kann beispielsweise in einem Kulturmedium erfolgen, welches vorzugsweise mit autologem oder homologem Serum angereichert ist.

Die Zellen können insbesondere während eines Zeitraumes von 14 Tagen bis 30 Tagen, insbesondere 17 Tagen bis 24 Tagen, bevorzugt 19 Tagen bis 21 Tagen, kultiviert werden.

Die prognostische Beurteilung wird in einer bevorzugten Ausführungsform anhand eines mittels der Transkriptomanalyse gewonnenen Transkriptomprofils und/oder eines auf das Transkriptomprofil zurückgehenden Genexpressionsprofils vorgenommen.

In einer weitergehenden Ausführungsform werden/wird das Transkriptomprofil und/oder das auf das Transkriptomprofil zurückgehende Genexpressionsprofil mit einem Transkriptomprofil und/oder Genexpressionsprofil desselben Zelltyps verglichen, welche/welches indikativ oder spezifisch bzw. charakteristisch für eine erfolgreiche Geweberegeneration, erfolgreiche Implantation und/oder erfolgreiche Transplantation und/oder für das Vorliegen von zellulärer Potenz sind/ist.

Alternativ oder in Ergänzung zur vorhergehenden Ausführungsform werden/wird das Transkriptomprofil und/oder das auf das Transkriptomprofil zurückgehende Genexpressionsprofil mit einem Transkriptomprofil und/oder Genexpressionsprofil desselben Zelltyps verglichen, welche/welches indikativ oder spezifisch bzw. charakteristisch für eine erfolglose bzw. wenig aussichtsreiche Geweberegeneration, erfolglose bzw. wenig aussichtsreiche Implantation und/oder erfolglose bzw. wenig aussichtsreiche Transplantation und/oder für das Fehlen von zellulärer Potenz sind/ist.

Anhand der in den beiden vorangegangenen Ausführungsformen erwähnten indikativen oder spezifischen bzw. charakteristischen Transkriptom- und/oder Genexpressionprofile ist mit besonderem Vorteil eine zuverlässige(re) Prognose eines möglichen Geweberegenerations-, Implantations- bzw. Implantat- und/oder Transplantationserfolges oder - anders ausgedrückt - eines möglichen Geweberegenerations-, Implantations- bzw. Implantat- und/oder Transplantationsversagens möglich.

Das für eine erfolgreiche Geweberegeneration, erfolgreiche Implantation und/oder erfolgreiche Transplantation und/oder das Vorliegen von zellulärer Potenz indikative oder spezifische bzw. charakteristische Transkriptomprofil und/oder Genexpressionsprofil werden/wird bevorzugt mittels Auswertung von Transkriptomprofilen und/oder von auf die Transkriptomprofile zurückgehenden Genexpressionsprofilen desselben Zelltyps von Patienten bestimmt, bei denen die Geweberegeneration, Implantation und/oder Transplantation erfolgreich verlaufen ist, und/oder bei denen der Zelltyp potent gewesen ist.

Das für eine erfolglose bzw. wenig aussichtsreiche Geweberegeneration, erfolglose bzw. wenig aussichtsreiche Implantation und/oder erfolglose bzw. wenig aussichtsreiche Transplantation und/oder das Fehlen von zellulärer Potenz indikative oder spezifische bzw. charakteristische Transkriptomprofil und/oder Genexpressionsprofil werden/wird bevorzugt mittels Auswertung von Transkriptomprofilen und/oder von auf die Transkriptomprofile zurückgehenden Genexpressionsprofilen desselben Zelltyps von Patienten bestimmt, bei denen die Geweberegeneration, Implantation und/oder Transplantation erfolglos verlaufen oder fehlgeschlagen ist, und/oder bei denen der Zelltyp nicht potent gewesen ist.

Das in den vorhergehenden Ausführungsformen erwähnte indikative Transkriptomprofil und/oder Genexpressionsprofil werden/wird bevorzugt im Rahmen einer retrospektiven klinischen Nachbeobachtung bzw. Analyse von Therapieergebnissen bestimmt.

Weiterhin ist es bevorzugt, wenn die Bestimmung des indikativen Transkriptomprofils und/oder Genexpressionsprofils mittels eines Suchalgorithmus, bevorzugt computergestützten Suchalgorithmus, erfolgt. Dies kann unter Zuhilfenahme einer jeden (kommerziell) verfügbaren Auswertungssoftware für Transkriptomdaten erfolgen, wie beispielsweise im Anwendungsbeispiel dargestellt.

Zur Durchführung der Transkriptomanalyse wird in einer zweckdienlichen Ausführungsform RNA aus den Zellen isoliert. Hierzu werden die Zellen in der Regel in einer chemischen Umgebung lysiert, in der RNasen (Ribonukleasen) zügig denaturiert werden. Anschließend wird die RNA von den übrigen zellulären Bestandteilen wie beispielsweise DNA, Proteinen, Zuckern, Lipiden oder dergleichen getrennt. Die Isolierung der RNA kann auf einer Extraktion oder Aufreinigung beruhen. Zum Beispiel lässt sich RNA mittels der sogenannten Guanidiniumthiocyanatmethode mit anschließender Phenol-/Chloroform-Extraktion isolieren.

Die isolierte RNA kann einer Qualitäts- und/oder Quantitätsanalyse unterworfen werden. Eine qualitative Bestimmung der isolierten RNA kann beispielsweise mithilfe eines Photometers erfolgen, das zur Erstellung eines Nukleinsäure-Spektrums in der Regel zwischen 220 nm und 450 nm meist eine nur sehr geringe Probenmenge benötigt. Gemessen wird typischerweise zum einen das Absorptionsverhältnis von 260 nm/280 nm und zum anderen das Absorptionsverhältnis von 260 nm/230 nm. Das Absorptionsverhältnis 260 nm/280 nm sollte zwischen 1,8 und 2,0 liegen. Es lässt insbesondere Rückschlüsse auf Proteinkontaminationen zu. Das Absorptionsverhältnis 260 nm/230 nm sollte über 1,8 liegen und zeigt insbesondere Verunreinigungen mit Lösungsmitteln, Salzen sowie Proteinen an. Eine weitere geeignete Methode zur Qualitäts- und/oder Quantitätsanalyse stellt die elektrophoretische Analyse dar, bei welcher isolierte RNA kapillarelektrophoretisch in einem speziellen Chip unter Erhalt eines sogenannten RNA-Elektropherogramms aufgetrennt wird.

In einer weiteren Ausführungsform wird im Rahmen der Transkriptomanalyse nicht codierende RNA, insbesondere nicht codierende und nicht regulative RNA, entfernt.

Bevorzugt werden/wird im Rahmen der Transkriptomanalyse ribosomale RNA (rRNA) und/oder transfer-RNA (tRNA) entfernt. Auf diese Weise wird mit besonderem Vorteil eine Anreicherung codierender RNA und/oder regulativer RNA erzielt und die Durchführung der Transkriptomanalyse ohne störende Interferenz anderer RNA ermöglicht. Mit anderen Worten ist es bevorzugt, die Transkriptomanalyse ausschließlich anhand kodierender RNA und/oder regulativer RNA vorzunehmen. Besonders bevorzugt wird eine Abreicherung bzw. Depletion von rRNA vorgenommen.

Für die Entfernung von ribosomaler RNA (rRNA) stehen grundsätzlich Präparations-Kits diverser Hersteller zur Verfügung. Beispielsweise kann zur Entfernung von rRNA der RiboMinusTM Eukaryote Kit (Firma Life Technologies) verwendet werden, der auf der selektiven Entfernung häufig vorkommender großer ribosomaler RNA-Moleküle aus dem Pool der Gesamt-RNA basiert. Dies wird durch eine Hybridisierung dieser rRNAs mit sequenzspezifischen Biotinmarkierten Oligonukleotid-Sonden erreicht. Der hybridisierte Komplex wird dann durch mit Streptavidin beschichtete magnetische Kügelchen (Beads) immobilisiert und entfernt. Das rRNA-abgereicherte Produkt wird in der Regel anschließend noch konzentriert.

Nach Entfernung von nicht codierender und insbesondere nicht regulativer RNA, bevorzugt von ribosomaler RNA (rRNA) und/oder transfer-RNA (tRNA), kann (erneut) eine Qualitäts- und/oder Quantitätsanalyse durchgeführt werden. Insoweit wird vollständig auf die zuvor im Zusammenhang mit der isolierten RNA beschriebenen Qualitäts- und/oder Quantitätsanalysen Bezug genommen.

In einer besonders bevorzugten Ausführungsform wir die Transkriptomanalyse ausschließlich anhand von mRNA (messenger RNA bzw. Boten-RNA) durchgeführt. Bei der mRNA handelt es sich um prozessierte RNA, die unter anderem bereits das sogenannte Spleißen durchlaufen hat, d.h. im Gegensatz zur prä-mRNA oder natürlichen DNA keine In-trons (nicht codierende Abschnitte) mehr enthält.

In einer weiteren Ausführungsform umfasst die Transkriptomanalyse eine Fragmentierung von RNA, bevorzugt von mRNA. Die Fragmentierung kann mittels eines enzymatischen Verdaus, in der Regel mittels einer RNase (Ribonuklease) wie beispielsweise RNase III, und/oder physikalisch, beispielsweise mittels Ultraschall, erfolgen. Für das erfindungsgemäße Verfahren geeignete Fragmente können 30 bis 1000 Nukleotide umfassen. Bevorzugt wird die Fragmentierung nach Entfernung von rRNA durchgeführt.

Die Transkriptomanalyse umfasst in einer weiteren Ausführungsform die Durchführung einer reversen Transkription, d.h. die Umschreibung von RNA, insbesondere mRNA, in cDNA (komplementäre DNA). Die Umschreibung wird bevorzugt nach einer Fragmentierung der RNA vorgenommen. In der Regel erfolgt die Umschreibung mit Hilfe des Enzyms reverse Transkriptase. Das bei der reversen Transkription primär erhaltene Produkt ist ein cDNA-Strang, der mit dem ursprünglichen RNA-Strang hybridisiert ist. Letzterer kann sodann mittels RNase H abgebaut werden. In einem weiteren Schritt wird mittels einer DNA-abhängigen DNA-Polymerase (über einen Primer) ein zu dem schon bestehenden cDNA-Einzelstrang komplementärer DNA-Strang unter Erhalt doppelsträngiger cDNA synthetisiert.

In einer weiterführenden Ausführungsform umfasst die Transkriptomanalyse die Vervielfältigung bzw. Amplifizierung doppelsträngiger cDNA. Bevorzugt wird die cDNA mittels Polymerase-Kettenreaktion (PCR), insbesondere Emulsions-Polymerase-Kettenreaktion (Emulsions-PCR), vervielfältigt bzw. amplifiziert.

Mit Hilfe einer reversen Transkription und einer sich daran anschließenden Amplifizierung der im Zuge der reversen Transkription erhaltenen cDNA lassen sich mit besonderem Vorteil cDNA-Bibliotheken erstellen.

In zweckdienlichen Ausführungsformen kann vor der Vervielfältigung bzw. Amplifikation eine Größenselektion der doppelsträngigen cDNA mittels Polyacrylamid-Gelelektrophorese (PAGE) durchgeführt werden.

Die cDNA wird in einer weiteren Ausführungsform einer Sequenzierungsmethode unterworfen.

Bevorzugt umfasst die Transkriptomanalyse eine hybridisierungsbasiertes Mikro- oder Makro-Array-Methode, auch als DNA-Hybridisierungs-Array-Methode bezeichnet, oder eine sequenzbasierte Methode, vorzugsweise eine Sequenzierungsmethode der zweiten Generation.

Sowohl die Mikro- bzw. Makro-Array-Methode als auch die sequenzbasierte Methode ermöglicht jeweils die Ausdehnung der Genexpressionsanalyse auf einen genomweiten Ansatz, indem sie in einem Experiment die simultane Detektion der Expressionsunterschiede von mehreren Tausend Genen erlauben.

Die Mikro- oder Makro-Array-Methode ähnelt bezüglich ihres Funktionsprinzips herkömmlichen Hybridisierungstechniken der Molekularbiologie wie beispielsweise Northern- oder Southern-Blot-Analysen. Diese Verfahren nutzen die Eigenschaft von Nukleinsäuren, miteinander sequenzspezifisch zu hybridisieren. Unter Hybridisierung wird die nicht-kovalente Bindung zweier zueinander komplementärer Nukleinsäureeinzelstränge verstanden, die vorrangig auf der Ausbildung von Wasserstoffbrückenbindungen zwischen den heterozyklischen Basen der Nukleinsäuremoleküle beruht.

Bei der Mikro- oder Makro-Array-Methode bzw. der DNA-Hybridisierungs-Array-Methode werden Nukleinsäuren, deren Sequenzen bekannt sind, sogenannte Sonden, in großer Zahl und hoher Dichte, in der Regel mit Hilfe eines Roboters, auf einen Träger ortsaufgelöst aufgebracht und immobilisiert. Diese DNA-Hybridisierungs-Arrays werden daraufhin mit markierten Nukleinsäuren hybridisiert. Zur Markierung können zum Beispiel radioaktiv- oder fluoreszenmarkierte Nukleotide während der reversen Transkription von RNA, in der Regel mRNA, in cDNA eingebaut werden. Da eine Hybridisierung nur zwischen komplementären Nukleinsäuremolekülen erfolgt, ist die Intensität des gemessenen Signals proportional zur Häufigkeit der erfolgten Hybridisierungen. Da jede Position einer Sonde einem bestimmten Gen oder Genabschnitt entspricht, liefert die an dieser Position gemessene Signalintensität ein Maß für das relative Expressionsniveau dieses Gens. Abhängig von der Anzahl der verfügbaren Gensonden und der Dichte, mit der diese auf den Träger aufgebracht werden, können mittels derartiger Arrays mehrere Tausend Gene gleichzeitig analysiert werden.

Die Sequenzierungsmethoden der zweiten Generation basieren nicht mehr auf einer Auftrennung der DNA über Kapillarelektrophorese wie bei der sogenannten Sanger-Methode, sondern auf einer Kopplung von cDNA-Fragmenten an feste Träger und der komplementären Anbindung einzelner Nukleotide bzw. Oligonukleotide, wobei deren Anbindung mittels hochauflösender Kameras bestätigt wird.

In einer bevorzugten Ausführungsform des erfindungsgemäßen Verfahrens umfasst die Transkriptomanalyse eine Sequenzierungsmethode der zweiten Generation, ausgewählt aus der Gruppe umfassend Pyrosequenzierung, Sequenzierung durch Synthese (Sequencing-by-Synthesis) und Sequenzierung durch Ligation (Sequencing-by-Ligation).

Bei der Pyrosequenzierung werden DNA-Fragmente über Linkermoleküle, in der Regel in Form von Oligo-Peptid-Adaptern, an Beads (Kügelchen) hybridisiert (ein Fragment pro Bead). Die DNA-Fragmente werden sodann mittels einer Polymerase-Kettenreaktion (PCR) vervielfältigt. Hierzu kommen die Beads in eine Emulsion mit PCR-Reagenzien. Die infolge der Polymerase-Kettenreaktion neu gebildeten DNA-Kopien bleiben ebenfalls an den Beads hängen. Für die Sequenzierung werden die Beads anschließend auf geeigneten Titerplatten, bevorzugt PicoTiterplatten, verteilt, in deren Vertiefungen (Wells) sich Enzyme und Primer befinden, welche für die Durchführung der Sequenzierung benötigt werden. Nacheinander werden dann die vier Nukleotide Desoxyadenosintriphosphat (dATP), Desoxyguanosintriphosphat (dDTP), Desoxycytidintriphosphat (dCTP) und Desoxythymidintriphosphat (dTTP) zugegeben. Bei jedem Nukleotid-Einbau wird Pyrophosphat freigesetzt, welches als ATP beispielsweise das Enzym Luziferase anregt, Luziferin in Oxiluziferin und Licht umzuwandeln. Die entsprechenden Vertiefungen der Titerplatten leuchten auf. Da pro Sequenzierschritt nur ein Nukleotid zugegeben wird, lässt sich auf diese Weise anhand des Signals die Sequenz der DNA-Fragmente bestimmen.

Bei der Sequenzierungsmethode "Sequenzierung durch Synthese" (Sequencing-by-Synthesis) werden reversible Terminator-Nukleotide eingesetzt. Die zu sequenzierenden DNA-Fragmente werden an die Glasoberfläche einer Flusszelle gebunden und mittels einer Polymerase-Kettenreaktion (PCR) vervielfältigt. Die PCR-Kopien werden rund um das ursprüngliche DNA-Fragment fixiert, wodurch gruppenidentische Moleküle entstehen. Die Sequenzierung beruht - ähnlich der Sanger-Methode - auf reversiblen Terminator-Nukleotiden. Die Synthesereagenzien (Primer, DNA-Polymerase und vier unterschiedliche Fluoreszenzfarbstoff-markierte reversible Terminator-Nukleotide) werden auf die Flusszelle gegeben. Wird an einem DNA-Fragment eines der vier Terminator-Nukleotide angefügt, blockiert das Fluorophor die weitere Synthese. Die Reaktion stoppt kurz, Farbstoff und Terminator-Nukleotid werden abgespalten, und das Leuchtsignal wird dokumentiert, ehe eine neue Runde beginnt.

Bei der Sequenzierungsmethode "Sequenzierung durch Ligation" (Sequencing-by-Ligation) erfolgt nach einer Emulsions-Polymerase-Kettenreaktion (Emulsions-PCR) an Beads (Kügelchen) die eigentliche Sequenzierreaktion. In einer ersten Runde (von insgesamt fünf) werden neben universellen Sequenzierprimern (Länge n) eine Mischung von vier verschiedenen Oktamer-Oligonukleotiden in die Reaktion gegeben. An den Positionen 1 und 2 dieser Oktamere sitzen definierte Basen (vier von 16 möglichen Dinukleotid-Paarungen; in den fünf Runden werden alle 16 möglichen Dinukleotide eingesetzt), die durch einen von vier Fluoreszenzfarbstoffen codiert sind. Das passende Oktamer-Oligonukleotid hybridisiert an das PCR-Fragment und wird an den ebenfalls hybridisierten Sequenzierprimer anligiert. Das Fluoreszenzsignal wird gemessen und der Farbstoff zusammen mit den letzten drei Nukleotiden entfernt. Diese Schritte werden mehrmals wiederholt, je nach DNA-Länge (bei 30-35 Basen wären das 6-7 Runden, wobei im nächsten Zyklus die Basen 6/7, danach 11/12 abgefragt werden). Schließlich werden sämtliche anligierten Oligo-Primer-Konstrukte wieder entfernt (Reset). Eine neue Runde startet mit einem neuen Sequenzierprimer der Länge n-1 und vier anderen Fluoreszenz-markierten Dinukleotiden. Jetzt werden also in der ersten Zyklusrunde die Basen n-1 und 1, dann Base 5/6, 10/11 usw. identifiziert. Nach drei weiteren Runden (Primer n-2, n-3 und n-4) liegt die Sequenz vor, jede Base wurde von zwei verschiedenen Oligonukleotiden überprüft.

Bezüglich eines Überblicks über die derzeit etablierten Hochdurchsatzsequenzierungsmethoden wird auf die Publikationen von Niedringhaus et al. (Landscape of Next-Generation Sequencing Technologies, Anal. Chem. 2011, 83, 4327 - 4341) und Hurd et al. (Advantages of next-generation sequencing versus the microarray in epigenetic research, BRIEFINGS IN FUNCTIONAL GENOMICS AND PROTEOMICS. VOL 8. NO. 3. 174-183) sowie auf den Artikel von Hollricher (Hochleistungs-Sequenzieren, Laborjournal 2009, 4, 44 - 48) Bezug genommen, deren Offenbarungsgehalt jeweils bezüglich der darin beschriebenen Sequenzierungsmethoden durch ausdrückliche Bezugnahme zum Inhalt der vorliegenden Beschreibung gemacht wird.

Die Transkriptomanalyse umfasst in einer weiteren Ausführungsform eine Assemblierung bzw. ein Zusammenfügen der sequenzierten cDNA bzw. cDNA-Fragmente. Auf diese Weise lassen sich Rückschlüsse auf funktionelle oder evolutionäre Verwandtschaften und damit auf die ursprüngliche Sequenz ziehen. Die Assemblierung kann mittels entsprechender, dem Fachmann geläufigen bioinformatischen Methoden durchgeführt werden.

In einer weiteren Ausführungsform werden die assemblierten cDNA-Fragmente einer Genannotation unterworfen, die eine Identifizierung von informationstragenden Sequenzen, insbesondere von differentiell regulierten Genen, erlaubt. Zweckmäßigerweise wird die Genannotation durch bioinformatische Methoden unterstützt, mittels derer Muster bzw. Profile und Zusammenhänge entdeckt und in Beziehung zu bekanntem Wissen, insbesondere über metabolische und regulatorische Netzwerke, gebracht werden können. Grundsätzlich erfordert die Analyse dieser Daten eine Normalisierung vor der eigentlichen Verarbeitung, zum Beispiel durch Cluster-Verfahren. Liegen die Daten in Form von Quotienten aus Messwerten über ein Behandlungsexperiment und ein Referenzexperiment vor, wird in der Regel eine Normalisierung durch Logarithmusbildung erreicht. Andere Normalisierungen basieren beispielsweise auf der Vektornorm, der Rangordnung, der einheitlichen Varianz oder dem sogenannten Z-Score. Bei letzterem handelt es sich um ein Verfahren zur Entscheidung, ob ein bestimmter Wert signifikant unter, am oder über einem Mittelwert liegt. Dabei ist ein negativer Wert ein Indikator für kleinere und ein positiver Wert ein Indikator für größere Werte als ein Mittelwert. Die Analyse der Standardabweichung liefert dann noch die Signifikanz dieser Abweichung. Für eine Visualisierung dieser Daten stehen diverse Softwaresysteme zur Verfügung, die in der Regel einerseits eine Strukturierung der Daten anhand verschiedener funktioneller Kategorien ermöglichen, sowie andererseits eine Visualisierung gemäß der erfolgten Kategorisierung. Die Funktionszuordnung bzw. Kategorisierung kann grundsätzlich auf der Grundlage verfügbarer Annotationen in Verbindung mit bekannten Suchalgorithmen erfolgen oder aber durch eine Kombination aus verfügbaren Annotationen und individuell aufgefundenen Suchalgorithmen. Grundlage derartiger Suchalgorithmen bilden meist Differenz-Analysen, bei welchen das Genexpressionsmuster einer zu untersuchenden Probe mit Referenzproben, die einen bestimmten pathophysiologischen Phänotyp abbilden, verglichen wird. Anhand dieser Daten können dann Suchalgorithmen programmiert werden, die speziell auf die zu identifizierenden Zellzustände abgestimmt sind.

Des Weiteren wird die Verwendung der Transkriptomanalyse, insbesondere von Transkriptomprofilen und/oder von auf ein Transkriptom zurückgehenden bzw. auf einem Transkriptom basierenden Genexpressionsprofilen, zur prognostischen Beurteilung der Geweberegenerationsfähigkeit und/oder der zellulären Potenz und/oder der Erfolgsaussichten einer Implantation, insbesondere Zellimplantation, und/oder Transplantation offenbart. Um unnötige Wiederholungen zu vermeiden, wird bezüglich weiterer Merkmale und Vorteile vollständig auf die bisherige Beschreibung Bezug genommen.

Weitere Merkmale und Vorteile der Erfindung ergeben sich aus den nachfolgend beschriebenen Ausführungsformen anhand von Figuren, Figurenbeschreibungen, eines Beispiels sowie der Unteransprüche. Hierbei können einzelne Merkmale der Erfindung alleine oder in Kombination miteinander verwirklicht sein. Die beschriebenen Ausführungsformen dienen lediglich zur Erläuterung und zum besseren Verständnis der Erfindung und sind in keiner Weise einschränkend zu verstehen.

### FIGURENBESCHREIBUNGEN

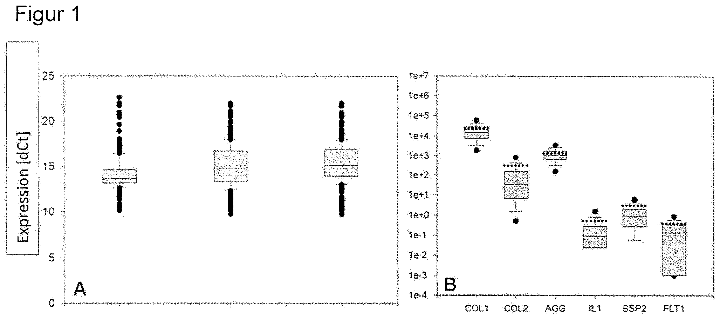

Figur 1 zeigt mittels qRT-PCR (quantitative real time PCR; quantitative Echtzeit-PCR) ermittelte Expressionswerte von GAPDH (A) und sechs ausgewählten Marker-Genen (B). Der Box-Plot zeigt für jedes Gen den Bereich von 25% - 75% (graue Box), den Bereich von 5% / 95% (horizontale Linien über- und unterhalb der Box), den Median (Linie innerhalb der Box) sowie Ausreißer (schwarze Punkte). A: Die GAPDH-Expressionswerte wurden in 3 wiederholenden Messungen (jeweils unter Verwendung der gesamten 422 Patientenproben) auf die gesamt-mRNA-Expression normiert. Die Unterscheide zwischen den drei Datensätzen sind statistisch nicht signifikant (einfache ANOVA), was auf die Zuverlässigkeit der qRT-PCR-Methode hindeutet. B: Die Expression der sechs Marker-Gene ist im Verhältnis zu GAPDH als negativer dCt-Wert dargestellt. Die Daten wurden im Rahmen einer Routinequalitätskontrolle zum Zeitpunkt der Ernte der einschichtigen Zellkulturen direkt vor der Besiedelung des Trägers (Novocart® Basic) erhoben. Der für jeden Patienten erhaltene mRNA-Expressionswert wurde auf den cDNA-Standard des betreffenden Patienten (gemäß den Herstellerangaben des cDNA-Synthese-Kits) normiert, so dass eine direkte Vergleichbarkeit der Expressionsdaten für jedes der sechs ausgewählten Gene gewährleistet ist. COL1: COL1A2, Kollagen Typ I Alpha-2-Kette; COL2: COL2A1, Kollagen Typ IIb Alpha-1-Kette; AGG: ACAN, Aggrecan; IL1: IL1-β, Interleukin-1β; BSP2: Knochen-Sialoprotein 2; FLT-1: vaskulärer endothelialer Wachstumsfaktor-Rezeptor 1.

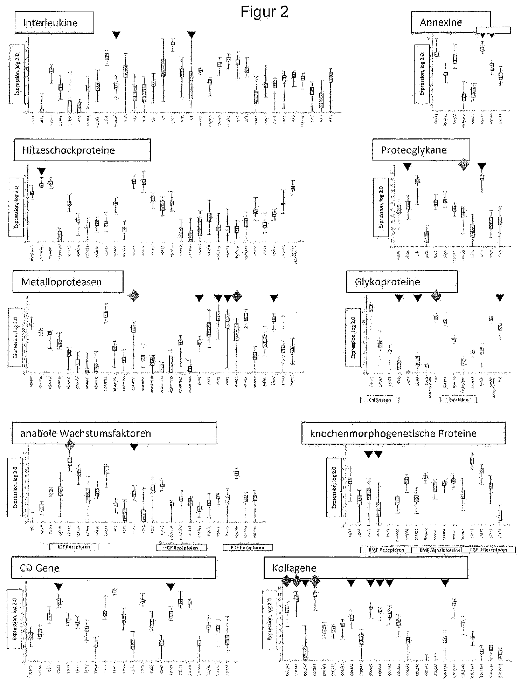

Figur 2 zeigt die Verteilung der Expressionslevel verschiedener Proteinfamilien in kultivierten humanen Chondrozyten. Die in logarithmischem Maßstab dargestellten RPKM Expressionswerte wurden für jede der 20 Proben im Rahmen einer Transkriptomanalyse erhoben. Der Box-Plot zeigt für jedes Gen einen Bereich von 25% - 75% (graue Box), den Median (Linie innerhalb der Box) sowie den gesamten Bereich (horizontale Linien über- und unterhalb der Box). Dreiecke markieren Gene, denen positive (z.B. FGF-2) bzw. anabole Eigenschaften (z.B. ACAN) in Bezug auf Knorpel zugeordnet werden können. Rauten markieren Gene, denen unerwünschte (z.B. Kollagen I) bzw. negative oder katabole Eigenschaften (z.B. Interleukin-1, ADAM-TS5) zugeschrieben werden. Genbezeichnungen entsprechen der NCBI Nomenklatur.

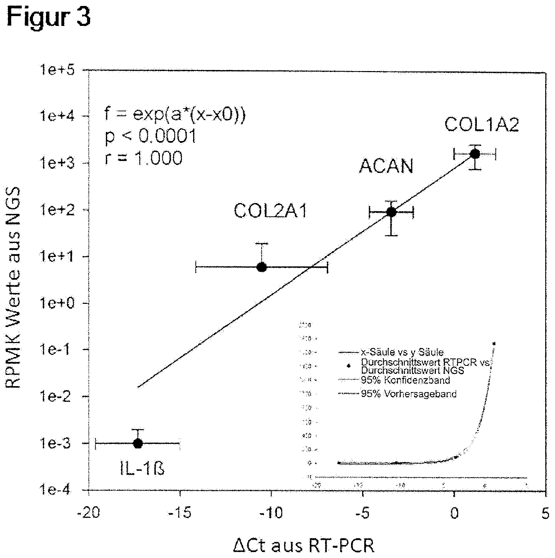

Figur 3 zeigt die Korrelationsanalyse zwischen qRT-PCR und RNA-Sequenzierung (NGS, Next Generation Sequencing) basierend auf den Expressionsdaten von COL1A2, COL2A1, ACAN und IL-1ß. Die Expressionswerte für COL1A2, COL2A1, ACAN und IL-1ß wurden zum einen mittels konventioneller qRT-PCR aus 422 Proben (vgl. Tabelle 1) und zum anderen mittels NGS-basierter RNA-Sequenzierung aus 20 Proben erhalten. ΔCt-Werte und RPKM-Expressionswerte sind logarithmisch dargestellt. Das Nebenbild zeigt die Auftragung der RPKM-Expressionswerte vor ihrer logarithmischen Konvertierung, wobei ihre sehr gute Korrelation noch deutlicher erkennbar wird.

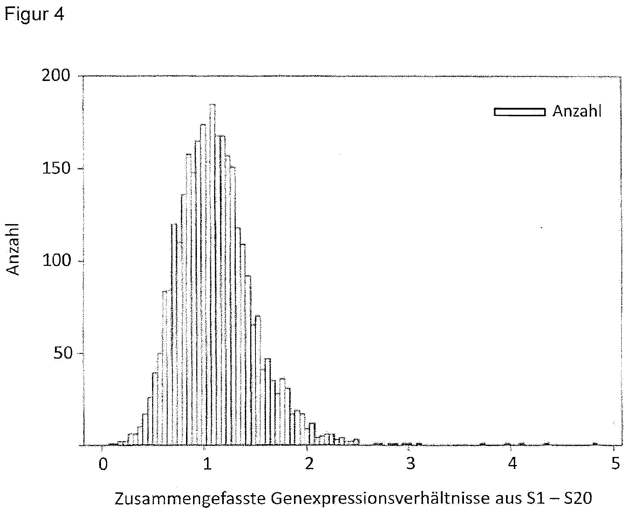

Figur 4 zeigt die Verteilung der Zahlenwerte für die Genexpressionsverhältnisse der am stärksten exprimierten 3114 Gene in Form eines Histogramms. Die Zahlenwerte wurden aus dem Verhältnis der gemittelten RPKM-Werte jedes transkribierten Gens aus der Gruppe mit guten klinischen Ergebnissen (S1 bis S10) zum Mittelwert aus der Gruppe mit Implantatversagen (S11 bis S20) erhalten. Zahlenwerte > 1 korrelieren mit guten klinischen Ergebnissen. Zahlenwerten < 1 korrelieren mit Implantatversagen. Jeder Balken repräsentiert 1/100 der gesamten erfassten Bereichs (kleinster Zahlenwert: 0,006; größter Zahlenwert: 4,9).

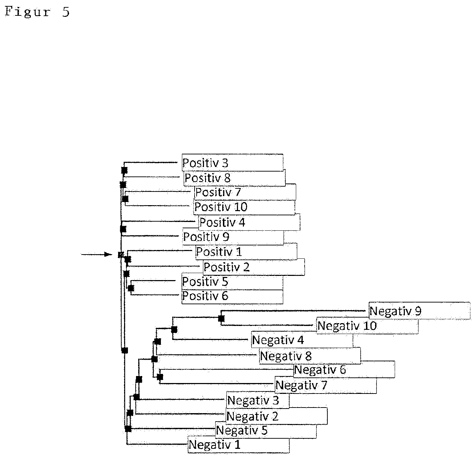

Figur 5 zeigt das hierarchische Clustering anhand der genomweiten Expressionsdaten der 20 Proben (S1 bis S20). Das Clustering basiert auf einer Pearson-Korrelation zwischen den Expressionswerten aller Gene einer Probe unter Anwendung des Neighbor-Joining-Algorithmus. Die Proben sind gemäß dem klinischen Ergebnis der Implantation bei den betreffenden Patienten als "Positiv" oder "Negativ" bezeichnet. Während die Varianz unter den Negativ-Proben erheblich höher ist als unter den Positiv-Proben, kann eine klare Trennung zwischen den zugrundeliegenden klinischen Resultaten verzeichnet werden. Diese Trennung deutet darauf hin, dass die klinischen Resultate - positiv bzw. negativ verlaufene Transplantation - einem transkriptomischen Phänotyp der für die Implantation herangezogenen Zellen zugeordnet werden können.

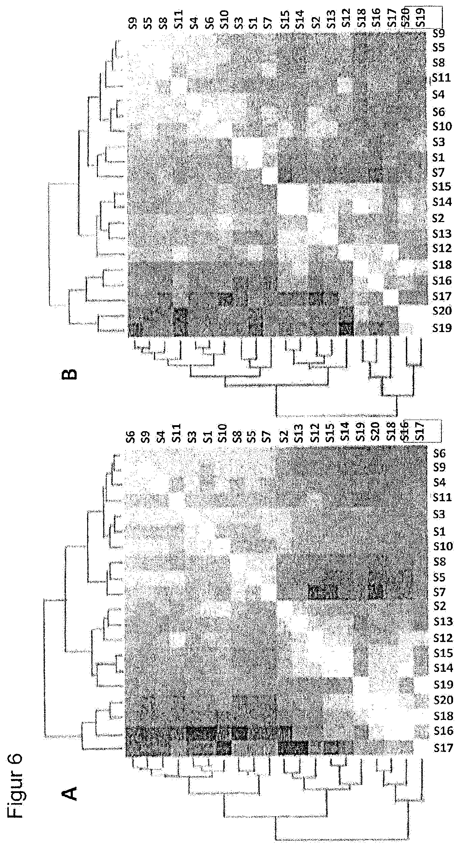

Figur 6 zeigt zwei Heatmap-Auswertungen A und B der Pearson-Korrelationsclusteranalyse aus den RPMK-Zahlenwerten der Proben S1 bis S20. Die Werte wurden gemäß ihrer Verwandtschaft angeordnet, wobei eng miteinander verwandte Expressionsmuster nahe beieinander liegen. A: Berücksichtigt wurden die kompletten Gene mit allen ihren Exonen. B: Die Exone wurden einzeln und unabhängig von der mRNA-Struktur analysiert. Abgesehen von S2 und S11 konnte mit dieser Auswertung eine Trennung der Proben S1 bis S20 gemäß dem zugrundeliegenden klinischen Verlauf erzielt werden (S1 - S10: positiver Verlauf; S11 - S20: Implantatversagen).

### BEISPIELTEIL

### 1. Material und Methoden

### 1.1 Aufbau der Studie

Es wurde eine retrospektive Erhebung über erste Ergebnisse, Nebenwirkungen und Veränderungen des Ausgangszustands bezüglich Schmerzen, Funktion und Schwellungen bei einer Patientenpopulation wie unten definiert durchgeführt. Es wurden weitere Analysen vorgenommen, um die Einflüsse von Patienten, Herstellung und zellbiologischen Eigenschaften auf Sicherheit und Patientenergebnisse zu untersuchen. Die klinischen und chirurgischen Verfahrensweisen, darunter Indikationen und Rehabilitation wurden in Standardarbeitsanweisungen (SOP) definiert. Chirurgen wurden in den chirurgischen Techniken zur Knorpelgewinnung und Implantationschirurgie geschult, bevor sie das Implantat das erste Mal anwendeten. Nachdem die Patienten ihre informierte Zustimmung erteilt hatten, wurde die Behandlungsindikation durch Arthroskopie bestätigt. Im betroffenen Gelenk wurden aus der Fossa intercondylaris (einem nicht belasteten Bereich) zwei bis drei Knorpel-Knochen-Stücke unter Verwendung eines sterilen und validierten Standardtrephine (Aesculap AG, Tuttlingen, Deutschland, Stanzdurchmesser: 4 mm) entnommen.

### 1.2 Klinische Datenerfassung

Der Chirurg jedes Patienten wurde aufgefordert, einen Datenerfassungsbogen auszufüllen, der die Krankheitsgeschichte (Ätiologie), demografische Grunddaten (Alter, Geschlecht) und Zeitraum zwischen chirurgischem Eingriff und letztem Patientenkontakt umfasste. Gemeinsam mit dem Patienten wurden Schmerzintensität, Funktion und Schwellungen auf einer 10-Punkteskala sowohl beim Gespräch vor dem Eingriff als auch nach dem Eingriff bewertet. Die Ergebnisbewertungsskala wurde aus der Visuellen Analogskala (VAS) modifiziert. Höhere Werte zeigen bessere Ergebnisse an (d. h. 10 bedeutet "keine Schmerzen", "keine Schwellung", "keine Funktionsbeeinträchtigung"). Diese Ergebnisabstufung mit 10-Punkten wurde von jedem Chirurgen bei einem Patientengespräch durchgeführt und wurde als Frühindikator für den weiteren klinischen Verlauf herangezogen. Die beteiligten Chirurgen wurden auch aufgefordert, alle auftretenden Nebenwirkungen und ebenso eine anschließend erforderliche Behandlung anzugeben. Der Fragebogen untersuchte nicht, ob die Patienten auf ein früheres arthroskopisches oder ein anderes chirurgisches Knorpelreparaturverfahren ungenügend ansprachen. Im Extremfall konnte die unerwünschte Wirkung ein Implantatversagen durch Nichteinheilung oder Ausreißen darstellen.

### 1.3 Population der Studie

Die Erhebung wurde an einundsechzig Zentren in Deutschland durchgeführt, in denen 433 Patienten behandelt wurden. Es wurden für insgesamt 422 Patienten (97,4 %) Daten zurückgemeldet. Die übrigen 11 Patienten wurden aus der weiteren Studie sowie den RNA-Analysen ausgeschlossen. Unter den 422 Patienten waren 140 Frauen und 282 Männer. Ihr mittleres Alter betrug 33,4 Jahre (min: 14,7 Jahre, max: 66.3 Jahre). Die Mehrzahl der primären Knorpeldefekte (Schäden von Grad III bis IV gemäß der Klassifikation der International Cartilage Repair Society (ICRS)) wurden am medialen Femurkondylus des Knies (68,5 %) festgestellt, gefolgt vom lateralen Kondylus (14,9 %), dem retropatellaren Bereich (10, 2 %), der Trochlea (5 %) und der Tibia (0,2 %). Bei sechs Patienten (1,4 %) wurde der Defekt am Talus festgestellt. Die Mehrzahl der Patienten wies einen einzigen Defekt auf (93 %). Die Defektgröße schwankte zwischen 1 und 20 cm². Die mittlere Größe betrug 5,9 cm². Die mittlere Dauer der Patientennachsorge betrug 6,9 Monate. Bei insgesamt 83 Patienten war mindestens ein Jahr seit dem chirurgischen Eingriff bis zum Tag des letzten vom Chirurgen eingetragenen Besuchs verstrichen (Tabelle 1).

**Tabelle 1: Population der Studie (422 Patienten)**

| | | absolute Zahl | Prozent |
|---|---|---|---|
| Geschlecht | Männlich | 282 | 66.8% |
| | Weiblich | 140 | 33.2% |
| Alter | Mittelwert | 33.4 | |
| | SD | 10.0 | |
| | Bereich | 14.7 - 66.3 | |
| Dauer der Patientennachsorge in Tagen | Mittelwert | 210 | |
| | SD | 185 | |
| | Bereich | 0 - 921 | |
| Diagnose | chronische Schädigung | 24 | 5.7% |
| | degenerativer Defekt | 144 | 34.1% |
| | Osteochondrosis dissecans | 123 | 29.1% |
| | traumatischer Defekt | 137 | 32.5% |
| Lokalisierung des Primärdefekts | Laterale Femurkondyle | 63 | 14.9% |
| | Mediale Femurkondyle | 289 | 68.5% |
| | Patella | 43 | 10.2% |
| | Talus | 6 | 1.4% |
| | Tibia | 1 | 0.2% |
| | Trochlea | 21 | 5.0% |
| Primärdefektgröße (cm²) | Mittelwert | 5.9 | |
| | SD | 3.1 | |
| | Bereich | 1 - 20 | |
| Anzahl behandelter Defekte | 1 | 391 | 92.7% |
| | 2 | 29 | 6.9% |
| | fehlende Daten | 2 | 0.5% |

Tabelle 1 zeigt die Population der Studie mit den entsprechenden Patientenmerkmalen. Die Merkmale sind absoluten Patientenzahlen und wo zutreffend prozentualen Anteilen zugeordnet. Eine Mehrfachdiagnose war bei einem Patienten möglich, wobei die Defekte mehr als eine Region betrafen.

### 1.4 Kultivierung

Nach der Entfernung von Knochen, mineralisiertem und oberflächlichem Knorpel wurden Chondrozyten aus dem verbliebenen Knorpel durch mechanische und enzymatische Extraktion isoliert, eingestellt nach Standardverfahren und in einer gemäß Good Manufacturing Practice (GMP) zugelassenen Einrichtung (TETEC AG, Reutlingen, Deutschland) als primäre Kultur in vitro vermehrt. Die Schritte umfassten, nach Zellisolation, eine Zellvermehrung in Kulturmedium, das mit autologem Serum angereichert war, Ernte durch Trypsinierung und Besiedlung in das Gerüst (Novocart Basic), was 21 Tage nach Arthroskopie zur Knorpelgewinnung stattfand. Das Gerüst wurde mit einer mittleren Zelldosis von 1,36 x 106 Zellen pro cm² besiedelt. Typischerweise wurden Zellen direkt aus der Primärkultur aufgebracht. Unter besonderen Umständen (z. B. Krankheit des Patienten) wurden Zellen vor einer Anwendung kryokonserviert und aus Sekundärkulturen verabreicht (weniger als 10 % der Fälle).

### 1.5 Hochdurchsatz-RNA-Sequenzierung

20 RNA-Proben aus den im vorhergehenden Abschnitt beschriebenen Chargen wurden analysiert, von denen zehn aus klinisch erfolgreichen Behandlungen kamen und zehn von Patienten mit negativen Ergebnissen erhalten wurden. Jeder Probe wurde eine eindeutige Barcodesequenz zugeordnet und Aliquote der mit Barcode versehenen Proben wurden je nach Patientenergebnis (positive Ergebnisse, negative Ergebnisse) in zwei Gruppen kombiniert.

Von jeder Probe wurde 1 bis 10 µg der gesamten RNA von Chondrozyten als Ausgangsmaterial verwendet. Die Gesamt-RNA wurde mit einem Agilent RNA 6000 Nano Chip (5067-1511) auf einem Gerät vom Typ Bioanalyzer 2100 bezüglich Quantität und Integrität analysiert. Eine Abreicherung bzw. Depletion ribosomaler RNA (rRNA) wurde unter Verwendung von 2-3 Durchgängen mit dem RiboMinusTMEukaryote Kit for RNA-Seq (A10837-08, life technologies) nach dem Protokoll des Herstellers durchgeführt. Eine Aufkonzentrierung erfolgte mit dem RiboMinus Concentration Module (K1550-05, life technologies). Die Effizienz der rRNA-Entfernung wurde in einem Durchgang eines Aliquots auf einem Agilent RNA 6000 Nano Chip (5067-1511) auf einem Gerät vom Typ Bioanalyzer 2100 geprüft und bestimmt, wobei sich eine vollständige Eliminierung von rRNA-Peaks zeigte.

Es wurde unter Verwendung des SOLiDTM Total RNA-Seq Kit (4445373, life technologies) nach dem Protokoll des Herstellers eine Bibliothek erstellt. Kurz gesagt, 10 bis 100 ng RNA nach Ribosomendepletion wurden zuerst durch 10 Minuten lange Verdauung bei 37 °C mit RNase III fragmentiert und erneut mit dem RiboMinus Concentration Module (K1550-05, life technologies) gereinigt und in 12 µl RNAse-freiem Wasser eluiert. Die Fragmentgröße wurde auf den optimalen Größenbereich für das SOLiD4-Gerät (150 bis 200 Nukleotide) geprüft, wobei wiederum ein Durchgang eines Aliquots auf einem Agilent RNA 6000 Nano Chip (5067-1511) auf einem Gerät vom Typ Bioanalyzer 2100 vorgenommen wurde.

Zum Aufbau von cDNA-Bibliotheken wurden die RNA-Fragmente strangspezifisch mit Adaptoren verknüpft und in doppelsträngige cDNA-Bibliotheken überführt, wobei eine reverse Transkription gefolgt von einer Größenselektion mit PAGE und Amplifikation durch PCR angewendet wurden. Während der PCR-Amplifikation wurden Barcodesequenzen eingefügt, wobei der 3'-Adaptorprimer einen spezifischen barcodespezifischen Overhang trug. Diese Methode vermeidet Barcodeverzerrungen, die berichtet wurden, wenn Barcodeadapter direkt mit der Bibliothek verknüpft werden. Erneut wurde die Fragmentgröße auf den Bereich von 150 bis 200 Nukleotide in einem Durchgang eines Aliquots auf einem Agilent DNA 1000 Chip (5067-1504) auf einem Gerät vom Typ Bioanalyzer 2100 geprüft.

Die Konzentrationsmessungen vom Bioanalyzer wurden zur Berechnung und äquimolaren Mischung von strichcodierten Proben S1-S10 (gute klinische Ergebnisse) und S11-S20 (negative klinische Ergebnisse) (jeweils Barcodes 1-10) verwendet. Es wurde für jede kombinierte Bibliothek eine Emulsions-PCR (emPCR) durchgeführt, jeweils in einem E20-Maßstab gemäß den Empfehlungen des Herstellers mit einer Bibliotheksendkonzentration von 0,5 pM. Das Brechen der Emulsion und das Waschen von Beads sowie die Anreicherung von Beads wurden von Hand nach dem Protokoll des Herstellers vorgenommen. Die Qualität und Quantität der Templat-Beads wurden in einem Durchgang der Workflow-Analyse (WFA) von 15 Millionen angereicherten Templat-Beads auf einem SOLiD4.0-Gerät gemessen. Das Signal-Rauschverhältnis betrug in beiden Bibliotheken 4 % und das Verhältnis P2/P1 betrug 100 %, was einen hohen Prozentanteil monoklonaler Beads anzeigt. 158 Millionen Templat-Beads der Bibliotheken wurden jeweils auf einem Quadfeld (1/4) eines SOLiD4,0-Trägers aufgebracht und durch Ligation vom Adaptor P1 sequenziert, so dass ein 50 bp Fragment-Lesedatensatz erhalten wurde.

### 1.6 Datenerstellung

Daten zu Nebenwirkungen und Patientenergebnissen wurden gesammelt und in ein Excel Spreadsheet eingetragen. Qualitätskontrolldaten vom Herstellen sowie Kovariablen (Merkmale zu Demographie, Patienten und Produktfreigabe) wurde in die gleiche Datenbank eingetragen. Alle eingegebenen Daten wurden auf Übereinstimmung geprüft.

### 1.7 Statistik und Kovariantenanalyse von Einzelparameterdaten

Summarische Statistik mit Mittelwerten, Standardabweichungen, Bereichen und Konfidenzintervallen wurde eingesetzt, um Summen von Nebenwirkungen, Wirksamkeitsergebnissen und Patientencharakteristiken aufzuzeigen. Die Freigabecharakteristiken jedes Produkts wurden mit den Erhebungsdaten verglichen, um Assoziationen zwischen Implantatproduktionsmerkmalen und Patientenergebnissen zu identifizieren. Es wurde eine Anzahl von Chi-Quadrat-Tests und Regressionsmodellen erstellt, um auf Assoziationen zwischen Herstellungs- oder Freigabecharakteristiken zu prüfen und die fünf Ergebnismessungen in der Patientensicherheitserhebung umfassten: implantatbezogene Nebenwirkungen (definiert als Zusammenfassung von Implantatversagen, Ablösung, Hypertrophie, Arthrofibrose, Adhäsionen, Chondromalazie, Gelenksinfektionen und Auftreten von freien Gelenkkörpern), Nachoperationen (jedweder Anlass) und Veränderungen des Ausgangszustands bezüglich Schmerzen, Funktion und Schwellungen. Regressionen zur Modellierung der Assoziation zwischen Biomarkern und einem interessierenden Ergebnis wurden sowohl für jeweils einen Biomarker und alle Biomarker gleichzeitig angepasst. Es wurde eine logistische Regressionsmodellierung angewendet, um den Einfluss der unabhängigen Kovarianten auf das Auftreten von implantatbezogenen Nebenwirkungen und dem Auftreten von Nachoperationen zu bestimmen. Es wurden Linearmodelle angewendet, um die Veränderungen des Ausgangszustands bezüglich Schmerzen, Funktion und Schwellungen zu modellieren. P-Werte wurden bei 0,05 als signifikant angenommen. Alle Regressionsmodelle umfassten den Zeitraum vom Eingriff bis zum letzten Arzttermin mit dem Patienten, da dies als ein bedeutender unabhängiger Vorhersagewert für die Ergebnisse vermutet wurde.

### 1.8 Statistische Analyse aller Transkriptomdaten

Lesesequenzen wurden mit der Whole Transcriptome Analysis Pipeline der BioScope 1.32 Software (Applied Biosystems) auf das menschliche Genom abgebildet (UCSC Version HG 19). Ausgehend von den Anmerkungen der UCSC refSeq (heruntergeladen am 7. November 2010) wurden RPKM-Werte der Expression für Gene berechnet. In Kürze, RPKM-Werte (Reads Per Kilobase exon model per Million mapped) stellen die Anzahl an sequenzierten Abschnitten, die sich auf die Exons einer gegebenen Transkription abbilden, normalisiert durch die Sequenzierungstiefe pro Probe (Gesamtabschnittszahl) und die Länge (bp) aller Exons dar. Diese Werte wurden für die Anfangsbestimmung verwendet. Die von Bullard et al. (J.H. Bullard, E. Purdom, K.D. Hansen, S. Dudoit, Evaluation of statistical methods for normalization and differential expression in mRNA-Seq experiments. BMC Bioinformatics 11: 94 (2010)) beschriebene Skalennormalisierungsmethode wurde eingesetzt, um probenspezifische technische Verzerrungen zu eliminieren und die erhaltenen Expressionswerte wurden auf einen logarithmischen Maßstab abgebildet. Proben wurden mit dem Neighbor-Joining-Algorithmus (N. Saitou and M. Nei, The neighbor-joining method: a new method for reconstructing phylogenetic trees. Mol. Biol. Evol. 4 (4): 406-425 (1987)) geklustert, der auf der Pearson-Korrelationsdistanz zwischen ihren ganzen Transkriptomexpressionsprofilen basiert. Das nichtparametrische Rank-Product-Verfahren (R. Breitling, P. Armengaud, A. Amtmann, P. Herzyk, Rank products: a simple, yet powerful, new method to detect differentially regulated genes. FEBS Letters 573 (1): 83-92 (2004)) wurde eingesetzt, um auf differentielle Expression zu prüfen, woraus pfp-Werte (Prozent falsch positiver Werte, ein Maß vergleichbar mit FDR-korrigierten p-Werten) erhalten werden, und Transkripte mit pfp < 0,05 und einem Absolutwert des Foldchange größer als 0,9 (d. h. verdoppelte bzw. halbierte Expression) wurden als signifikante differentielle Expression betrachtet. Es wurden die differentiell exprimierten proteinkodierenden Transkripte verwendet, um auf eine Anreicherung spezifischer Funktionskategorien zu prüfen (Überrepräsentationsanalyse, hypergeometrischer Test, auf Falschbestimmungsrate korrigierte p-Werte). Für alle Analysen wurde die von Mayday beschriebene Technik (F. Battke, S. Symons, K. Nieselt, Mayday -Integrative analytics for expression data. BMC Bioinformatics 11 (1): 121 (2010)) verwendet. Mit dem Integrative Genomics Viewer IGV (J.T. Robinson, H. Thorvaldsdottir, W. Winckler, M. Guttman, E.S. Lander, G. Getz, J.P. Mesirov, Integrative genomics viewer. Nat. Biotech. 29: 24-26 (2011)) wurden die Abschnitte einzelner Gene von Interesse im Detail untersucht.

### 2. Ergebnisse

### 2.1 Veränderungen des Ausgangszustands bei den Patientenergebnissen

Vor einem chirurgischen Eingriff und bei nachfolgenden Patientenbesuchen bestimmten die Ärzte Gelenkschmerzen, Funktion und Schwellungen auf einer 10-Punkteskala, wobei höhere Werte bessere Ergebnisse anzeigen. Patienten, die sowohl zu Fragen vor dem Eingriff als auch nach dem Eingriff Antworten gaben, erreichten signifikante Verbesserungen gegenüber dem Ausgangszustand (p < 0,0001, Wilcoxon Signed-Rank Test) bei allen drei Messungen. Die mittlere Zeitdauer seit dem chirurgischen Eingriff betrug 6,9 Monate (Bereich: 2 bis 30 Monate), was ein merklich kürzerer Zeithorizont ist, als er bei den meisten anderen Zusammenfassungen von ACI-Ergebnissen angegeben ist. Die retrospektive Studie steuerte wesentliche Aspekte zum vorliegenden Wissen zur Reparatur von Gelenkknorpeln bei. Bei der Untergruppe von 83 Patienten mit mindestens 12 Monaten seit ihrem chirurgischen Eingriff wurde berichtet, dass sie im Mittel stärkere Veränderungen bei allen drei Ergebnismessungen aufwiesen als die gesamte Patientenpopulation (Tabelle 2).

**Tabelle 2: Individuelle Ergebnismessung**

| | | Alle Patienten (N=422) | | | | | Untersuchungszeitraum >1 Jahr (N=83) | | | | |
|---|---|---|---|---|---|---|---|---|---|---|---|
| Ergebnismessung | | Min | Mittel | Max | SD | N | Min | Mittel | Min | SD | N |
| Schmerz | präoperativ | 1 | 3.4 | 10 | 1.3 | 412 | 2 | 3.4 | 9 | 1.2 | 82 |
| | postoperativ | 1 | 7.0 | 10 | 2.0 | 382 | 2 | 7.2 | 10 | 1.9 | 80 |
| | Veränderung | -5 | 3.8 | 8 | 2.1 | 376 | 2 | 3.9 | 8 | 2.1 | 80 |
| Schwellung | präoperativ | 1 | 5.3 | 10 | 2.4 | 411 | 1 | 5.1 | 10 | 2.4 | 82 |
| | postoperativ | 1 | 7.7 | 10 | 1.9 | 382 | 4 | 8.1 | 10 | 1.6 | 80 |
| | Veränderung | -7 | 2.5 | 9 | 2.6 | 375 | -3 | 3.1 | 8 | 2.2 | 80 |
| Funktion | präoperativ | 1 | 4.2 | 10 | 1.8 | 412 | 2 | 4.4 | 10 | 2.0 | 82 |
| | postoperativ | 2 | 7.3 | 10 | 1.8 | 380 | 2 | 7.8 | 10 | 1.6 | 80 |
| | Veränderung | -7 | 3.2 | 8 | 2.3 | 374 | -7 | 3.6 | 8 | 2.4 | 80 |

### 2.2 Auftreten von Nebenwirkungen

Tabelle 3 zeigt das Auftreten von berichteten Nebenwirkungen. Das Auftreten von Implantatversagen betrug 3,1 % bei der gesamten Patientenpopulation und 6 % bei der Untergruppe von Patienten, bei denen mindestens 12 Monate seit ihrem Eingriff verstrichen waren. Allgemein waren die berichteten Fallzahlen von implantatbezogenen Komplikationen bei der gesamten Patientenpopulation gering. Ablösung (Delamination), Arthrofibrose und Hypertrophie wurden bei 1,7 %, 2,4 % bzw. 0,7 % beobachtet. Insgesamt 36 Patienten (8,5 %) erforderten eine Nachoperation und/oder Korrektur. Die häufigsten Nebenwirkungen, die bei Patienten berichtet wurden, die Nachoperationen erforderten, waren Implantatversagen (13, die durch Transkriptomanalyse weiter untersuchten zehn Proben stammten aus diesen Fällen), Ablösung (6), Arthrofibrose (7), Synovitis (7), Adhäsionen (5) und Schmerzen (6). Die Untergruppe von 83 Patienten, bei denen mindestens 12 Monate seit ihrem Eingriff verstrichen waren, wiesen eine Nachoperationsrate von 13,3 % auf. Die Mehrzahl der Nachoperationen wurde arthroskopisch durchgeführt.

**Tabelle 3: Berichtete Komplikationen**

| | Alle Patienten (N=422) | | | | Untersuch ungszeitraum >1 Jahr (N=83) | | | |
|---|---|---|---|---|---|---|---|---|
| Komplikation | Fälle | % | 95% | KI** | Fälle | % | 95% | KI** |
| implantatbezogene Komplikationen*** | | | | | | | | |
| Implantatversagen | 13 | 3.1% | 1.7% | 5.2% | 5 | 6.0% | 2.0% | 13.5% |
| Delamination | 7 | 1.7% | 0.7% | 3.4% | 2 | 2.4% | 0.3% | 8.4% |
| Hypertrophie | 3 | 0.7% | 0.2% | 2.1% | 1 | 1.2% | 0.0% | 6.5% |
| Arthrofibrose | 10 | 2.4% | 1.1% | 4.3% | 2 | 2.4% | 0.3% | 8.4% |
| Adhesionen | 7 | 1.7% | 0.7% | 3.4% | 2 | 2.4% | 0.3% | 8.4% |
| freie Gelenkkörper | 1 | 0.2% | 0.0% | 1.3% | 1 | 1.2% | 0.0% | 6.5% |
| tiefe (Gelenk-)Infektion | 3 | 0.7% | 0.2% | 2.1% | 0 | | - | |
| Chondromalacia | 2 | 0.5% | 0% | 1.7% | 0 | | - | |

| Weitere Komplikationen | | | | | | | | |
|---|---|---|---|---|---|---|---|---|
| Effusion | 32 | 7.6% | 5.2% | 10.5% | 6 | 7.2% | 2.7% | 15.1% |
| Schmerz | 29 | 6.9% | 4.7% | 9.7% | 7 | 8.4% | 3.5% | 16.6% |
| Synovialitis | 14 | 3.3% | 1.8% | 5.5% | 3 | 3.6% | 0.8% | 10.2% |
| Hämatom/Hämarthrose | 7 | 1.7% | 0.7% | 3.4% | 5 | 6.0% | 2.0% | 13.5% |
| Versteifung | 1 | 0.2% | 0.0% | 1.3% | 1 | 1.2% | 0.0% | 6.5% |
| oberflächliche Infektion | 2 | 0.5% | 0.0% | 1.7% | 0 | | - | |

### 2.3 Assoziation zwischen Ergebnissen und unabhängigen Risikofaktoren

Die Größe aller behandelten Defekte zeigte keine Korrelation mit einem der gemessenen Patientenergebnisse. Für die Veränderung des Ausgangszustands bei der Funktion des Patienten wurde eine signifikante Assoziation mit dem Patientenalter gefunden (d. h. bei niedrigerem Alter zeigten sich im Mittel größere Verbesserungen, p = 0,004, nicht gezeigt). Tabelle 4 gibt die Resultate von Chi-Quadrat-Tests zur Assoziation der Lage des primären Defekts auf das Auftreten von implantatbezogenen Nebenwirkungen an. Es wurde gefunden, dass sowohl das Auftreten von implantatbezogenen Nebenwirkungen wie von Nachoperationen weitgehend unabhängig von der Lage des primären Defekts ist, mit Ausnahme von patellaren Defekten. In jedem Fall war für einen Betroffenen die Wahrscheinlichkeit eines solchen Ereignisses größer, wenn der primäre Defekt an der Patella gelegen war (p < 0,0001).

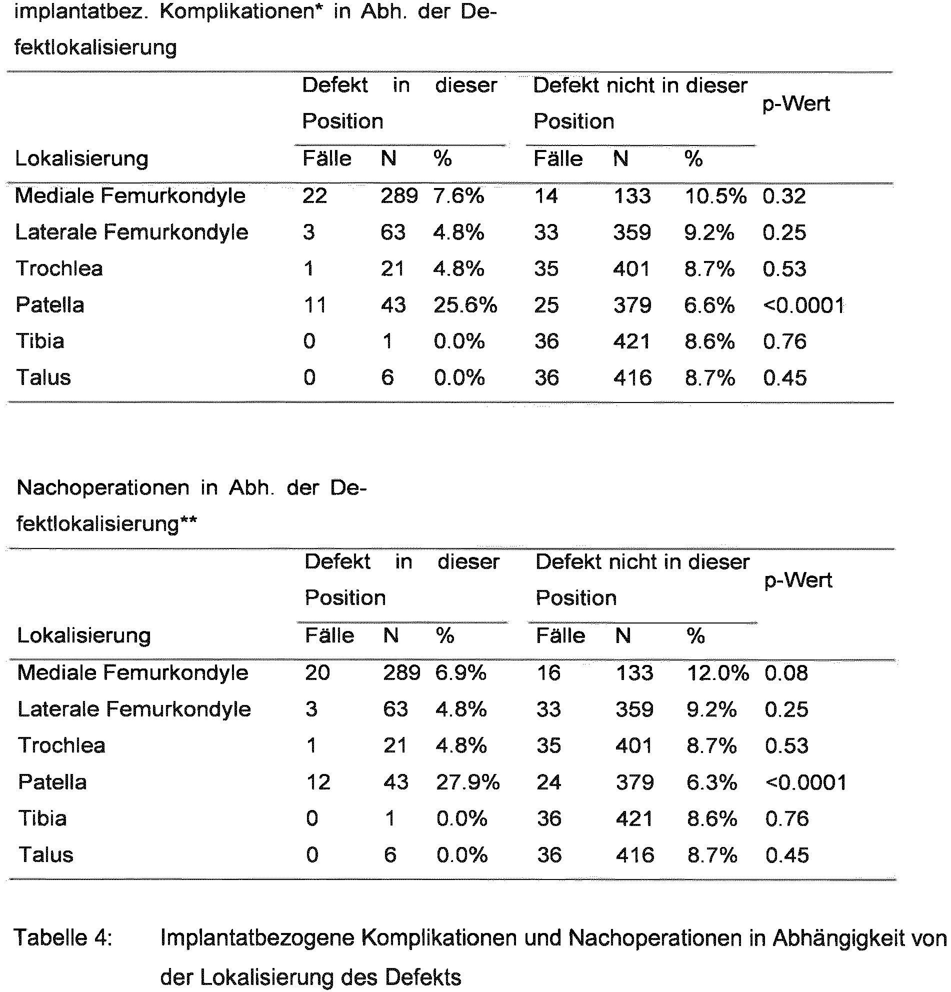

Tabelle 5 gibt die Resultate von Chi-Quadrat-Tests zur Assoziation zwischen der Klassifikation von Knorpelverletzungen und dem Auftreten von implantatbezogenen Nebenwirkungen und Nachoperationen an. Im Falle von implantatbezogenen Nebenwirkungen war für einen Betroffenen die Wahrscheinlichkeit eines solchen Ereignisses größer, wenn der Knorpeldefekt als degenerativ klassifiziert war (p = 0,005). Hingegen war für Patienten die Wahrscheinlichkeit eines solchen Ereignisses geringer, wenn ein durch Osteochondritis dissecans bedingter Knorpeldefekt vorlag (p = 0,04).

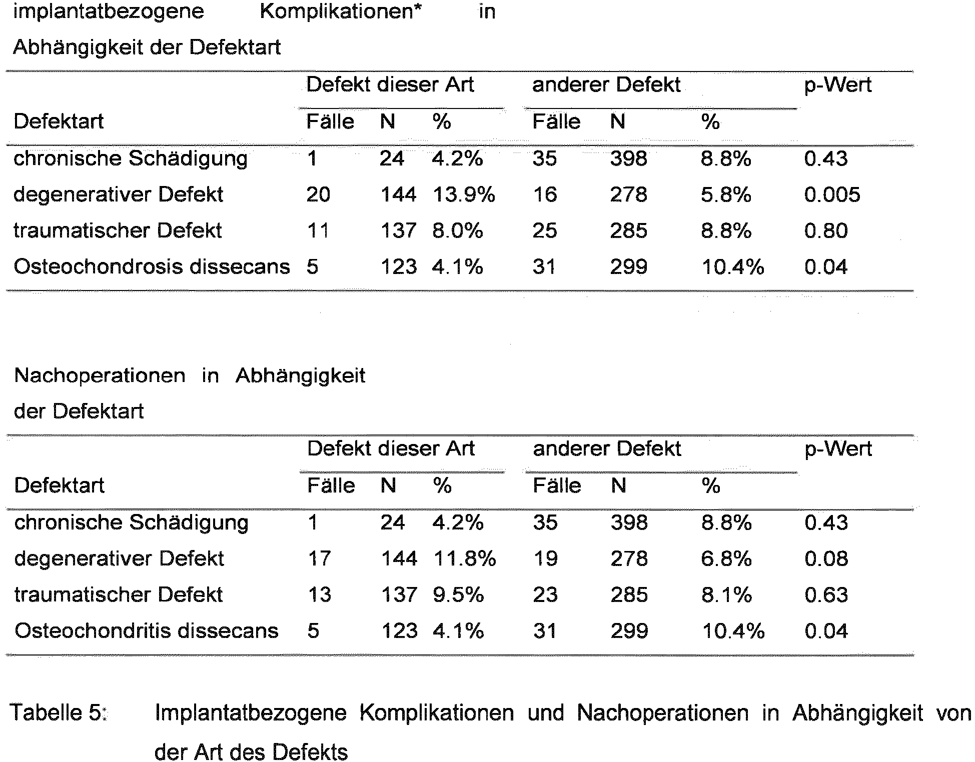

Tabelle 6 zeigt Assoziationen zwischen Patientenergebnissen und Zellkulturvariablen einerseits und mRNA-Expressionswerten von sechs ausgewählten Marker-Genen (vgl. Punkt 2.4.1) andererseits als multivariante p-Werte. Signifikante Zusammenhänge und grenzwertige Trends sind hervorgehoben. Eine temporäre Kryokonservierung der Zellen vor einer Implantation zeigte keine Assoziation mit einem verstärkten Auftreten von implantatbezogenen Komplikationen (IBK) oder mit einer anderen der Ergebnismessungen (Nachoperation NO, Schmerz, Funktion, Schwellung). Bezüglich der Zellviabilität wurden die gleichen Resultate beobachtet. Allerdings war eine geringe Anzahl an verabreichten Zellen signifikant mit einer Nachoperation (NO) assoziiert (p = 0,06; Tabelle 6).

**Tabelle 6: Zusammenhang zwischen Patientenergebnissen und Zellkulturvariablen bzw. mRNA-Expressionswerten von sechs ausgewählten Marker-Genen**

| Zellkulturvariablen | IBK | NO | Schmerz | Funktion | Schwellung |
|---|---|---|---|---|---|
| Kryokonservierung | 0.98 | 0.98 | 0.59 | 0.78 | 0.13 |
| Zellviabilität bei Ernte | 0.56 | 0.13 | 0.55 | 0.38 | 0.76 |
| Zellzahl (log 10) | 0.30 | 0.06 | 0.47 | 0.37 | 0.36 |
| | | | | | |

| PCR-Expressionsmessung | | | | | |
|---|---|---|---|---|---|
| Aggrecan | 0.14 | 0.44 | 0.61 | 0.50 | 0.62 |
| BSP-2 | 0.11 | 0.14 | 0.76 | 0.78 | 0.90 |
| Kollagen I | 0.49 | 0.70 | 0.53 | 0.79 | 0.27 |
| Kollagen II | 0.08 | 0.08 | 0.42 | 0.90 | 0.29 |
| Interleukin-1β | 0.08 | 0.20 | 0.26 | 0.19 | 0.24 |
| FLT-1 | 0.02 | 0.03 | 0.21 | 0.67 | 0.81 |

### 2.4 Expressionsdaten

### 2.4.1 Spezifische Genexpression

Mittels quantitativer Echtzeit-PCR (qRT-PCR) wurde die Expression von sechs ausgewählten Marker-Genen bestimmt: Aggrecan (ACAN), einem integralen Bestandteil der extrazellulären Knorpelmatrix; Kollagen Typ I (COL1A2), das üblicherweise nicht in Knorpelgewebe vorkommt; knorpelspezifisches Kollagen Typ II (COL2A1); Interleukin-1β (IL-1β), ein inflammatorisches Zytokin; FLT-1, eine Isoform eines vaskulären endothelialen Wachstumsfaktor-Rezeptors; und Knochen-Sialoprotein BSP-2, ein Knochenwachstumsfaktor. Die beiden letztgenannten wurden deshalb ausgewählt, da Komplikationen insbesondere aus einer Vaskularisierung des implantierten Knorpelgewebes bzw. aus einer Bildung von Osteophyten innerhalb des implantierten Knorpelgewebes resultieren können.

Die Expressionswerte (dCt) dieser sechs Marker-Gene sind in der Figur 1 dargestellt und zeigen signifikante Unterschiede. Die stabilsten mRNA-Level, die im Bereich einer Größenordnung zwischen allen 422 Proben variieren, wurden für Aggrecan gefunden. Die größten Variationen, im Bereich von drei Größenordnungen, bei einem insgesamt sehr niedrigen Detektionslevel, traten bei FLT-1 auf. Für Kollagen Typ I beispielsweise konnten durchschnittlich 1000 mRNA-Transkripte pro Zelle berechnet werden, während im Falle von FLT-1 lediglich 10 mRNA-Transkripte pro 1000 Zellen berechnet wurden. Zum Vergleich enthalten beispielsweise in vitro kultivierte humane venöse Endothelzellen mindestens 500-mal mehr FLT-1 mRNA-Transkripte.

Trotz dieser Variationen konnten dennoch signifikante oder zumindest grenzwertige Korrelationen zwischen einigen dieser Marker-Gene und dem klinischen Resultat beobachtet werden (Tabellen 6 und 7). So konnte eine signifikante Assoziation zwischen implantatbezogenen Komplikationen (IBK) und erhöhten FLT-1 Expressionslevel in vermehrten Chondrozyten festgestellt werden (p = 0,02, Tabelle 6). Lediglich ein grenzwertiger Zusammenhang konnte dagegen für erhöhte IL-1β Level und erniedrigte Kollagen Typ II Level (beide p = 0,08, Tabelle 6) bestimmt werden. Unter Berücksichtigung aller Marker-Gene bei den Rechenmodellen konnte jedoch für IL-1β (p = 0,02, Tabelle 6) und FLT-1 (p = 0,08, Tabelle 6) ein signifikanter bzw. grenzwertiger Zusammenhang in Bezug auf IBK gefunden werden.

Gemäß Tabelle 7, welche für die sechs Marker-Gene neben den Regressionskoeffizienten die multivarianten p-Werte unter Berücksichtigung der verstrichenen Zeit seit dem Eingriff zeigt, konnte die Expression keines der Marker-Gene in Bezug auf Schmerz, Funktion und Schwellung als signifikant eingestuft werden. Die Koeffizienten für die Patientenergebnisse IBK und NO (Nachoperation) wurden mittels logistischer Regression, die Koeffizienten für Schmerz, Funktion und Schwellung mittels linearer Regression berechnet. Die p-Werte (zweite Reihe) indizieren die Signifikanz des betreffenden Zusammenhangs, wobei signifikante Zusammenhänge und grenzwertige Trends hervorgehoben sind.

**Tabelle 7: Multivariate Analyse des Zusammenhangs zwischen Patientenergebnissen und der Zeit seit dem Eingriff bzw. den mRNA-Expressionswerten von sechs ausgewählten Marker-Genen.**

| Variable | IBK | NO | Schmerz | Funktion | Schwellung |
|---|---|---|---|---|---|
| Zeit seit Eingriff | 1.057 | 1.064 | 0.0084 | 0.055 | 0.060 |
| | *0.037* | *0.017* | *0.644* | *0.0049* | *0.0059* |

| PCR Expressionsmessung | | | | | |
|---|---|---|---|---|---|
| Aggrecan | 1.141 | 0.962 | 0.105 | -0.099 | 0.087 |
| p= | *0.492* | *0.836* | *0.343* | *0.397* | *0.506* |
| BSP-2 | 1.086 | 1.078 | -0.018 | -0.013 | -0.0085 |
| p= | *0.161* | *0.177* | *0.607* | *0.730* | *0.837* |
| Kollagen I | 0.901 | 0.973 | 0.015 | 0.049 | 0.057 |
| p= | *0.426* | *0.835* | *0.849* | *0.564* | *0.556* |
| Kollagen II | 1.095 | 1.112 | -0.034 | 0.015 | -0.054 |
| p= | *0.166* | *0.107* | *0.347* | *0.698* | *0.216* |
| Interleukin-1β | 1.151 | 1.112 | -0.044 | -0.049 | -0.054 |
| p= | *0.029* | *0.083* | *0.197* | *0.177* | *0.179* |
| FLT-1 | 1.106 | 1.102 | -0.033 | -0.0096 | -0.0041 |
| p= | *0.062* | *0.070* | *0.222* | *0.739* | *0.901* |

### 2.4.2 Genomweite Expression

Die Gesamtheit der erfassten Expressionsdaten umfasste etwa 30.000 Gene mit einer großen Vielfalt an Exondaten und ergab mehr als 200 Millionen Einzeldatenabschnitte. Für die Gruppe S1-10 (gute klinische Ergebnisse) wurden 42.716.671 Abschnitte (75,3 %) eindeutig abgebildet, 3.828.740 (6,7 %) waren Spanning-Exon-Junctions, 3.791.685 der Exon-Junctions waren bekannt und 37.055 waren neu. Für die Gruppe S11-20 (Implantatversagen) waren insgesamt 43.429.958 Abschnitte (76,0 %) eindeutig abgebildet, 3.991.606 Abschnitte (7,0 %) waren Spanning-Exon-Junctions, 3.958.041 der Exon-Junctions waren bekannt und 33.565 waren neu. Die abgebildeten Abschnitte wurden zur Berechnung von RPKM-Werten (Reads Per Kilobase per Million mapped reads-Werten) für jedes in der UCSC refSeq-Datenbank (heruntergeladen 07.11.2010) enthaltenen Exons mit Bioscope 1.3 verwendet. Eine erste Erhebung dieser RPKM wurde unter Verwendung von Genfamilien vorgenommen, wobei Familienmitglieder berücksichtigt wurden, die "typisch" für Chondrozyten sind, zum Beispiel Kollagene (mit Kollagenen der Typen II, IX und XI als "Knorpelkollagene") oder Proteoglykane (mit Aggrecan (ACAN) als typischer Vertreter) (Fig. 2). Die Daten sind graphisch dargestellt, wobei die Standardabweichungen die Exonvariabilität angeben. Außerdem erstreckten sich die Expressionswerte über Größenordnungen und konnten nur in logarithmischem Maßstab dargestellt werden, wie in Fig. 2. Die Resultate zeigen schon auf, dass der klassische "chondrozytische" Phänotyp die Resultate nicht dominiert. Eine weitere Analyse anderer Genfamilien, darunter beispielsweise IGF-bezogene oder PDGF/VEGF-bezogene Gene, bestätigt diese Vermutung. Eine Expression der Interleukin-Zytokine war diversifiziert. Die Expression dieser Wachstumsfaktoren wurde noch komplexer, wenn komplementäre Bindungsproteine oder die zugehörigen Rezeptoren heran-gezogen wurden. Gleichermaßen ist die Transkription der erweiterten Cluster von Metalloproteinasen sehr breit (Fig. 2).

### 2.5 Kalibrierung von Transkriptomdaten durch qRT-PCR

Um die Vergleichbarkeit von Daten bei den beiden Methoden abzuschätzen, wurde eine Korrelationsanalyse basierend auf den Expressionsdaten von COL1A2, COL2A1, ACAN und IL-1ß vorgenommen (vgl. Fig. 1). Diese Gene zeigen typische Expressionswerte in Chondrozyten im Bereich von sieben Zehnerpotenzen. Die Korrelation der Werte aus den beiden Verfahren war erfolgreich und ergab einen Korrelationskoeffizienten von 1,0 in einer Exponentialfunktion (Fig. 3).

### 2.6 Korrelation zwischen Transkriptomdaten und klinischen Ergebnissen

Um Informationen zu spezifischen Mustern zu erhalten, die (hypothetisch) mit den klinischen Ergebnissen in Relation stehen, wurden zwei Auswerteverfahren gewählt. In einem ersten Ansatz wurde das Expressionsverhältnis für den gemittelten RPKM-Wert jedes transkribierten Gens aus der Gruppe mit guten klinischen Ergebnissen (S1 bis S10) zum Mittelwert aus der Gruppe mit Implantatversagen (S11 bis S20) ermittelt. Dadurch wurde eine Liste von Zahlenwerten > 1 erhalten, die eine positive Korrelation mit guten Ergebnissen aufweisen, zu Zahlenwerten < 1, die eine positive Korrelation mit Implantatversagen aufweisen. Zahlenwerte nahe 1 weisen auf Gene hin, die sich zu den Ergebnissen neutral verhalten. Die Resultate wurden als Histogramm in Fig. 4 dargestellt. Das Histogramm enthält Werte für die am stärksten exprimierten 3114 Gene. 1803 Werte betragen > 1, 1311 Werte betragen < 1. Darunter betragen 62 Werte > 2, was eine zweifach stärkere Expression in der Gruppe mit guten Ergebnissen S1-10 bedeutet, und 70 Werte betragen < 0,5, was eine zweifach stärkere Expression in der Gruppe mit Implantatversagen bedeutet. Mit anderen Worten, stellen diese beiden Gencluster Kandidaten für eine eingeschränkte Liste von Qualitätsparametergenen für nachfolgende Studien dar. Diese Liste enthält keine regulatorischen RNAs.

In einem zweiten Ansatz wurde eine Korrelationsclusteranalyse aus den RPKM-Zahlenwerten durchgeführt: der Pearson-Korrelationskoeffizient wurde für jedes Probenpaar (S1-S20) bei den Genen berechnet und eine sogenannte "Heatmap" der Korrelationsmatrix mit der eingebauten Heatmap-Funktion aufgetragen. Die Hypothese hinter dieser Korrelation nimmt an, dass die Berechnung eine sortierte Liste für die 20 klinischen Ergebnisse ergibt, die die guten Ergebnisse von den klinischen Versagerfällen trennt, wenn die in den oben angeführten Daten skizzierten Relationen statistisch signifikant sind. Das Resultat ist in Fig. 5 dargestellt. In Fig. 6A wurden die Daten für die kompletten Gene mit allen ihren Exonen zusammengestellt. In Fig. 6B wurden die Exons einzeln und unabhängig von der mRNA-Struktur analysiert. Mit anderen Worten, in Fig. 6A ist alternatives Splicing vernachlässigt, während es in Fig. 6B dargestellt ist. Es stellte sich heraus, dass die Daten entsprechend ihrem Ursprung sortiert wurden, wobei die Gruppe S1-10 von der Gruppe S11-20 getrennt wurde. Ausnahmen sind die Probe S2, die in der Übergangszone zwischen den beiden Gruppen eingeordnet wurde, als sie auf Gene analysiert wurde, und weiter in die Versagergruppe abwanderte, als sie auf Exons analysiert wurde, und die Probe S11, die stabil im "falschen" Cluster verblieb. Insgesamt ergab die Heatmap eine korrekte "Vorhersage" des klinischen Ergebnisses bei 18 von 20 Fällen, oder mit einer Wahrscheinlichkeit von 90 %.

## Patentansprüche

1. In vitro-Verfahren zur prognostischen Beurteilung der Erfolgsaussichten einer Implantation und/oder Transplantation, **dadurch gekennzeichnet, dass** in autologen Chondrozyten die mRNA-Expressionswerte einer Gruppe bestehend aus COL2A1, IL-1β, FLT-1, BSP-2, COL1A2 und ACAN bestimmt werden.

2. In vitro-Verfahren nach Anspruch 1, **dadurch gekennzeichnet, dass** die mRNA mittels Echtzeit-PCR bestimmt wird.

3. In vitro-Verfahren nach Anspruch 1 oder 2, **dadurch gekennzeichnet, dass** es sich bei den Chondrozyten um Gelenkchondrozyten handelt.

4. In vitro-Verfahren nach einem der vorhergehenden Ansprüche, **dadurch gekennzeichnet, dass** es sich bei der Implantation um eine trägergestützte autologe Chondrozytenimplantation handelt.

5. In vitro-Verfahren nach einem der vorhergehenden Ansprüche, **dadurch gekennzeichnet, dass** es sich bei der Transplantation um eine autologe Chondrozytentransplantation handelt.

## Claims

1. In vitro method for prognostically assessing the prospects of success of an implantation and/or transplantation, **characterized in that** the mRNA expression values of a group consisting of COL2A1, IL-1β, FLT-1, BSP-2, COL1A2 and ACAN are determined in autologous chondrocytes.

2. In vitro method according to claim 1, **characterized in that** the mRNA is determined using real-time PCR.

3. In vitro method according to claim 1 or 2, **characterized in that** the chondrocytes are articular chondrocytes.

4. In vitro method according to any of the preceding claims, **characterized in that** the implantation is a support-assisted autologous chondrocyte implantation.

5. In vitro method according to any of the preceding claims, **characterized in that** the transplantation is an autologous chondrocyte transplantation.

## Revendications

1. Procédé in vitro pour l'évaluation pronostique des perspectives de succès d'une implantation et/ou d'une transplantation, **caractérisé en ce qu'**on détermine les valeurs d'expression d'ARNm d'un groupe constitué par COL2A1, IL-1β, FLT-1, BSP-2, COL1A2 et ACAN dans des chondrocytes autologues.

2. Procédé in vitro selon la revendication 1, **caractérisé en ce que** l'ARNm est déterminé par PCR en temps réel.

3. Procédé in vitro selon la revendication 1 ou 2, **caractérisé en ce que**, pour les chondrocytes, il s'agit de chondrocytes articulaires.

4. Procédé in vitro selon l'une quelconque des revendications précédentes, **caractérisé en ce qu'**il s'agit, pour l'implantation, d'une implantation de chondrocytes autologues supportés.

5. Procédé in vitro selon l'une quelconque des revendications précédentes, **caractérisé en ce qu'**il s'agit, pour la transplantation, d'une transplantation de chondrocytes autologues.
